# EUROPEAN PATENT SPECIFICATION

(11) **EP 3 930 789 B1**
(45) Date of publication and mention of the grant of the patent: **10.12.2025**
(21) Application number: 20710750.9
(22) Date of filing: 20.02.2020
(51) Int. Cl.: A61M 5/178, A61M 5/315

(54) **MEDICATION DELIVERY DEVICE WITH SENSING SYSTEM**
MEDIKAMENTENABGABEVORRICHTUNG MIT ERFASSUNGSSYSTEM
DISPOSITIF D'ADMINISTRATION DE MÉDICAMENT DOTÉ D'UN SYSTÈME DE DÉTECTION

(30) Priority: 27.02.2019 US 201962811228 P
(43) Date of publication of application: 05.01.2022
(60) Divisional application: 25213288.1
(73) Proprietor: Eli Lilly and Company, Indianapolis, IN 46285 (US)
(72) Inventor: CONNAUGHTON, Eoin Patrick, Indianapolis, Indiana 46206-6288 (US); LAURENZI, Brendan Francis, Indianapolis, Indiana 46206-6288 (US); LAWLOR, Vincent Patrick Thomas, Indianapolis, Indiana 46206-6288 (US); LI, Lin, Indianapolis, Indiana 46206-6288 (US); MURPHY, Patrick Kevin, Indianapolis, Indiana 46206-6288 (US); PSZENNY, Sean Matthew, Indianapolis, Indiana 46206-6288 (US)
(74) Representative: J A Kemp LLP
(86) International application number: PCT/US2020/018953
(87) International publication number: WO 2020/176316

(56) References cited:
- WO-A1-2018/013419
- WO-A1-2018/141571
- US-A1- 2016 287 807
- US-A1- 2018 369 494
- US-A1- 2019 022 328

## Description

### BACKGROUND

Patients suffering from various diseases must frequently inject themselves with medication. To allow a person to conveniently and accurately self-administer medicine, a variety of devices broadly known as pen injectors or injection pens have been developed. Generally, these pens are equipped with a cartridge including a piston and containing a multi-dose quantity of liquid medication. A drive member is movable forward to advance the piston in the cartridge to dispense the contained medication from an outlet at the distal cartridge end, typically through a needle.

In disposable or prefilled pens, after a pen has been utilized to exhaust the supply of medication within the cartridge, a user discards the entire pen and begins using a new replacement pen. In reusable pens, after a pen has been utilized to exhaust the supply of medication within the cartridge, the pen is disassembled to allow replacement of the spent cartridge with a fresh cartridge, and then the pen is reassembled for its subsequent use.

Such devices may have components that physically interact with one another to result in a state change or an action by the device. For example, the device may have a cap that is removed prior to delivery, a dose button that may be rotated to set a dose and/or actuated to deliver a dose, an "on" button that wakes the device, and so on.

The inventors have appreciated that switches can be used to detect the occurrence of such interactions. The inventors have also appreciated that some of these physical interactions may happen repeatedly. The inventors have thus recognized a need for a simple, low-cost switch that can be repeatedly opened and closed to detect such interactions.

US 2016/287807 A1 discloses a sensor assembly comprising a first rotary sensor part with a plurality of individual electrically conducting axial position sensor segments arranged in a circumferential pattern, and a second rotary sensor part arranged rotationally relative to the first portion and comprising a plurality of circumferentially arranged electrically interconnected axial position contacts adapted to be arranged in contact with the axial position sensor segments. The assembly further comprises actuator means for axially moving the axial position contacts between a connected and a dis-connected position, wherein the axial position contacts and the axial position sensor segments are arranged such that for a given rotational position at least two axial position contacts each are in contact with a different axial position sensor segment.

US 2018/369494 A1 discloses a rotary sensor assembly, comprising a first sensor part with a plurality of position sensor segments, and a second sensor part with a grounded contact in contact with a position sensor segment. For a current incremental rotational position a contact is in contact with an un-powered current-position sensor segment, with the neighbour segments being in a powered state. When the contact is rotated to a powered next-position position sensor segment, the next-position sensor segment becomes a new current-position sensor segment. Electronic circuitry is adapted to detect that the new current-position sensor segment is grounded and that the first and second sensor parts thereby have been rotated one increment relative to each other, and subsequently change the state of the former current-position sensor segment from un-powered to powered and the state of the new current-position sensor segment from powered to un-powered.

US 2019/022328 A1 discloses a dosage injection mechanism that produces a rotational motion when a drug injection pen dispenses a fluid. The apparatus also includes a button housing with at least part of a dosage measurement system disposed within the button housing. At least part of the dosage measurement system is coupled to receive the rotational motion from the dosage injection mechanism. The dosage measurement system includes one or more sensors positioned to output a signal in response to the rotational motion when the drug injection pen dispenses a fluid, and a controller coupled to the one or more sensors to receive the signal. A drug delivery control wheel of the drug injection pen is disposed between the body of the drug injection pen and the at least part of the dosage measurement system disposed in the button housing.

WO 2018/013419 A1 discloses a dose detection system for a medication delivery device. The dose detection system includes a dosing component attached to an actuator and rotationally and axially moveable relative to a coupling component attached to a dose setting member. The dose detection system further comprises a module including an electronic sensor operative to detect a relative rotation of the coupling component and the dosing component to detect a dose delivered by the medication delivery device.

WO 2018/141571 A1 discloses a dosage sensing module for a pen drug delivery device. The module comprises a power source unit, a processor unit and a dosage sensor unit with a first sensor part adapted to be fixed to a part not rotating during dose expelling and comprising a flexible printed circuit board sheet on which a pattern of individual electrical conductive sensor areas are arranged. The sensor unit further comprises a second sensor part adapted to be fixed to the piston rod to follow the rotation thereof during dose expelling. The second sensor part comprises individual structures that together with the conductive sensor areas are adapted to, upon relative rotational movement between the first and second sensor part, provide electrical signals to a processor unit indicative of the relative rotational position between the first and second sensor part.

### SUMMARY

The present disclosure relates to a medication delivery device having a switch comprising a conductive pad and a cantilevered arm that is movable relative to the conductive pad. Contact between the cantilevered arm and the conductive pad changes the state of the switch, such as closes the switch, and lack of contact between the cantilevered arm and the conductive pad changes the state of the switch, such as opens to switch. In some embodiments, the switch may be used to determine the amount of a dose of medication delivered by the medication delivery device.

The invention is defined in claim 1. Further aspects and preferred embodiments of the invention are defined in the dependent claims.

### BRIEF DESCRIPTION OF THE DRAWINGS

Additional embodiments of the disclosure, as well as features and advantages thereof, will become more apparent by reference to the description herein taken in conjunction with the accompanying drawings. The components in the figures are not necessarily to scale. Moreover, in the figures, like-referenced numerals designate corresponding parts throughout the different views.
FIG. 1 is a perspective view of a medication delivery device having a dose detection system according to aspects of the present disclosure.
FIG. 2 is a partially exploded perspective view of the medication delivery device of FIG. 1, showing a dose button having a support and a cover, where the cover is shown separated from the support.
FIG. 3 is a partially exploded side view of the medication delivery device of FIG. 1 showing the components of the dose detection system.
FIG. 4 is a cross-sectional view of the medication delivery device of FIG. 1.
FIG. 5 is a partial cutaway view of a proximal end of the medication delivery device of FIG. 1, showing components of the dose detection system.
FIG. 6 is an underside view of a portion of the dose button of FIG. 1, showing a printed circuit board held within the dose button cover.
FIG. 7 is an exploded view of the portion of the dose button shown in FIG. 6.
FIG. 8 is a perspective view of a flange of a dose detection system of a medication delivery device.
FIG. 9 is a top down view of the flange of FIG. 8.
FIG. 10 is a perspective view of a dose button support.
FIG. 11 is a top down view of the dose button support of FIG. 10.
FIG. 12 is a perspective view of a printed circuit board and sensor switch according to aspects of the present disclosure.
FIG. 13 is a perspective view of a cantilevered arm and base of the sensor switch of FIG. 12.
FIG. 14 is a side view of the cantilevered arm and base of FIG. 13.
FIG. 15 is a side view of the cantilevered arm of FIG. 12 positioned between two teeth of a flange.
FIG. 16 shows the cantilevered arm of FIG. 15 being pushed by one of the teeth of the flange during rotation of the flange.
FIG. 17 shows the cantilevered arm being pushed further by the tooth of the flange such that a portion of the cantilevered arm has moved toward and is in contact with a conductive pad, closing the switch.
FIG. 18 shows the cantilevered arm sliding over the tooth of the flange.
FIG. 19 shows the cantilevered arm interacting with the next adjacent tooth of the flange.
FIG. 20 is a perspective view of a switch design according to another embodiment.
FIG. 21 is a side view of the switch of FIG. 20.
FIG. 22 is a perspective view of a switch design according to another embodiment.
FIG. 23 is a side view of the switch of FIG. 22.
FIG. 24 is a perspective view of the switch of FIG. 22 interacting with a rotating flange.
FIG. 25 is a side view of the switch of FIG. 20 that shows a center of mass of the switch of FIG. 21.
FIG. 26 is a perspective view of a switch design according to another embodiment.
FIG. 27 is a side view of the switch of FIG. 26.
FIG. 28 is a perspective view of the switch of FIG. 26 interacting with a rotating flange.
FIG. 29 is a perspective view of a switch design according to another embodiment.
FIG. 30 is a perspective view of the switch of FIG. 29 mounted to a printed circuit board.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the invention is thereby intended.

The present disclosure relates to sensing systems for medication delivery devices.

In one aspect, the sensing system includes a switch having a conductive pad and a cantilevered arm that is moveable relative to the conductive pad. Contact between the cantilevered arm and the conductive pad changes to the first state of the switch, such as, closes the switch, and lack of contact between the cantilevered arm and the conductive pad changes to the second state of the switch, such as, opens the switch.

In another aspect, the switch is used for sensing of relative rotational movement between a dose-setting assembly and an actuator of the medication delivery device in order to determine the amount of a dose delivered by a medication delivery device. The sensed relative rotational movements are correlated to the amount of the dose delivered. By way of illustration, the medication delivery device is described in the form of a pen injector. However, the medication delivery device may be any device which is used to set and to deliver a dose of a medication, such as pen injectors, infusion pumps and syringes. The medication may be any of a type that may be delivered by such a medication delivery device.

Devices described herein may further comprise a medication, such as for example, within a reservoir or cartridge 20. In another embodiment, a system may comprise one or more devices including device 10 and a medication. The term "medication" refers to one or more therapeutic agents including but not limited to insulins, insulin analogs such as insulin lispro or insulin glargine, insulin derivatives, GLP-1 receptor agonists such as dulaglutide or liraglutide , glucagon, glucagon analogs, glucagon derivatives, gastric inhibitory polypeptide (GIP), GIP analogs, GIP derivatives, oxyntomodulin analogs, oxyntomodulin derivatives, therapeutic antibodies and any therapeutic agent that is capable of delivery by the above device. The medication as used in the device may be formulated with one or more excipients. The device is operated in a manner generally as described above by a patient, caregiver or healthcare professional to deliver medication to a person.

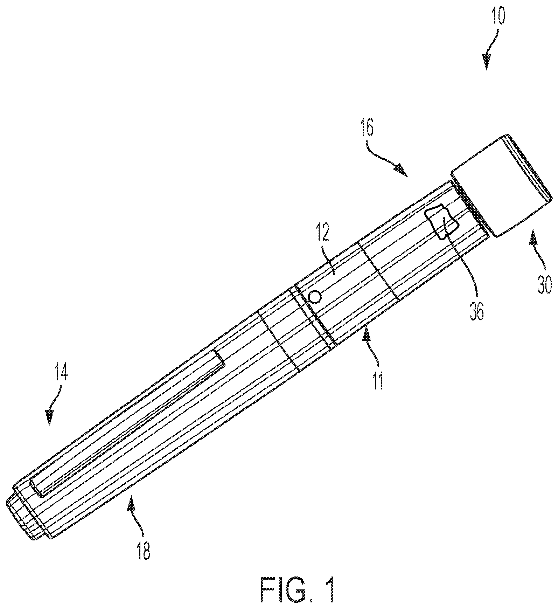

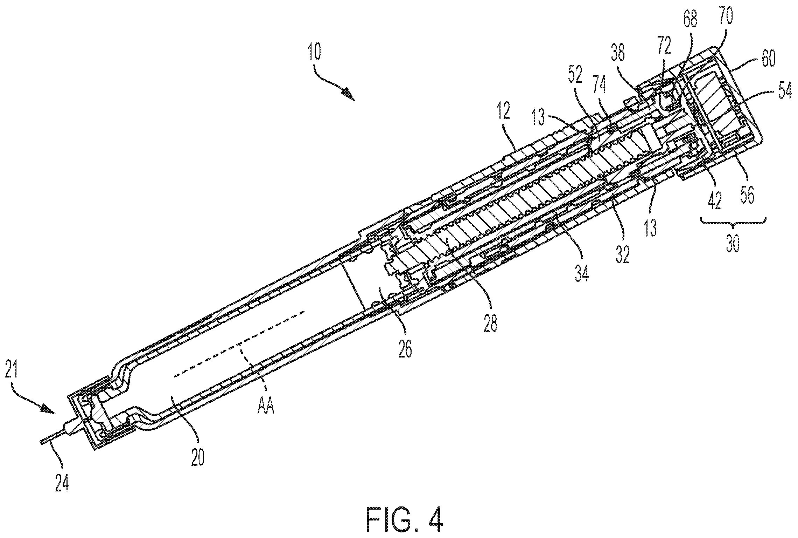

An exemplary medication delivery device 10 is illustrated in FIGS. 1-4 as a pen injector configured to inject a medication into a patient through a needle. Device 10 includes a body 11 that may comprise an elongated, pen-shaped housing 12 including a distal portion 14 and a proximal portion 16. Distal portion 14 may be received within a pen cap 18. Referring to FIG. 4, distal portion 14 may contain a reservoir or cartridge 20 configured to hold the medicinal fluid to be dispensed through the outlet 21 of the housing a dispensing operation. The outlet 21 of distal portion 14 may be equipped with an injection needle 24. In some embodiments, the injection needle is removable from the housing, while some embodiments include a needle fixed to the cartridge unit. In some embodiments, the injection needle is replaced with a new injection needle after each use.

A piston 26 may be positioned in reservoir 20. The medication delivery device may include an injecting mechanism positioned in proximal portion 16 that is operative to advance piston 26 toward the outlet of reservoir 20 during the dose dispensing operation to force the contained medicine through the needled end. The injecting mechanism may include a drive member 28, illustratively in the form of a screw, that is axially moveable relative to housing 12 to advance piston 26 through reservoir 20.

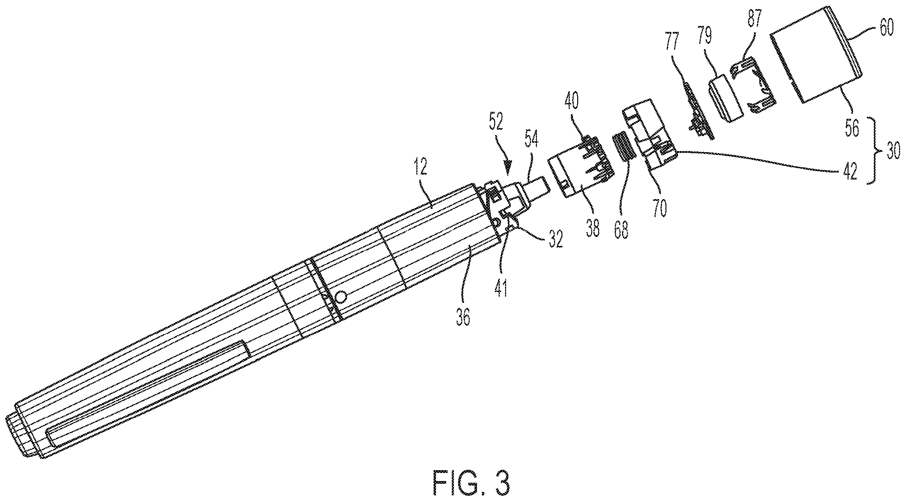

The device may include a dose-setting assembly coupled to the housing 12 for setting a dose amount to be dispensed by device 10. As best seen in FIGS. 3 and 4, in the illustrated embodiment, the dose-setting assembly includes a dose-setting screw 32 and a flange 38. The dose-setting screw 32 is in the form of a screw element operative to spiral (i.e., simultaneously move axially and rotationally) about a longitudinal axis AA of rotation relative to housing 12 during dose setting and dose dispensing. FIGS. 3 and 4 illustrate the dose-setting screw 32 fully screwed into housing 12 at its home or zero dose position. Dose-setting screw 32 is operative to screw out in a proximal direction from housing 12 until it reaches a fully extended position corresponding to a maximum dose deliverable by device 10 in a single injection. The extended position may be any position between a position corresponding to an incremental extended position (such as a dose setting a 0.5 or 1 unit) to a fully extended position corresponding to a maximum dose deliverable by device 10 in a single injection and to screw into housing 12 in a distal direction until it reaches the home or zero position corresponding to a minimum dose deliverable by device 10 in a single injection.

Referring to FIGS. 3 and 4, dose-setting screw 32 includes a helically threaded outer surface that engages a corresponding threaded inner surface 13 of housing 12 to allow dose-setting screw 32 to spiral (i.e. simultaneously rotate and translate) relative to housing 12. Dose-setting screw 32 further includes a helically threaded inner surface that engages a threaded outer surface of sleeve 34 (FIG. 4) of device 10. The sleeve 34 includes internal threads engaged with the external threads of drive member 28, which in turn, when sleeve 34 moving axially, drives drive member 28 axially to move the piston 26. The outer surface of dose-setting screw 32 includes dose indicator markings, such as numbers that are visible through a dosage window 36 to indicate to the user the set dose amount.

As mentioned above, in some embodiments, the dose-setting assembly further includes a tubular flange 38 that is coupled in the open proximal end of dose-setting screw 32 and is axially and rotationally locked to the dose-setting screw 32 by protrusions 40 received within openings 41 in the dose-setting screw 32. The protrusions 40 of the flange 38 can be seen in FIGS. 3, 8 and 9, and the openings 41 of the dose-setting screw 32 can be seen in FIG. 3.

As seen in FIGS. 3 and 4, delivery device 10 may include an actuator assembly having a clutch 52 and a dose button 30. The clutch 52 is received within the dose-setting screw 32, and the clutch 52 includes an axially extending stem 54 at its proximal end. The dose button 30 of the actuator assembly is positioned proximally of the dose-setting screw 32 and flange 38. Dose button 30 includes a support 42, and a cover 56. As will be discussed, the support 42 and cover 56 enclose electronics components used to store and/or communicate data relating to amount of dose delivered by a medication delivery device.

The support 42 of the dose button may be attached to the stem 54 of the clutch 52, such as with an interference fit or an ultrasonic weld, so as to axially and rotatably fix together dose button 30 and clutch 52.

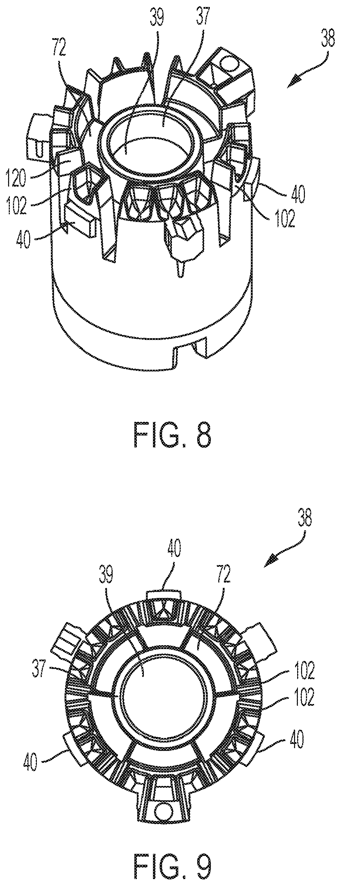

In some embodiments, a portion of the clutch may pass through a lumen 39 of the flange 38. The lumen 39 of the flange is best seen in FIGS. 8 and 9. The lumen 39 may, in some embodiments, serve to help center the clutch 52 in place.

Proximal face 60 of the dose button 30 may serve as a push surface against which a force can be applied manually, i.e., directly by the user to push the actuator assembly (dose button 30 and clutch 52) in a distal direction. A bias member 68, illustratively a spring, may be disposed between the distal surface 70 of support 42 and a proximal surface 72 of tubular flange 38 (FIGS. 8 and 9) to urge the support 42 of the actuation assembly and the flange 38 of the dose-setting assembly axially away from each other. Dose button 30 is depressible by a user to initiate the dose dispensing operation. In some embodiments, the bias member 68 is seated against this proximal surface 72 and may surround a raised collar 37 of the flange 38.

Delivery device 10 is operable in a dose setting mode and a dose dispensing mode. In the dose setting mode of operation, the dose button 30 is rotated relative to housing 12 to set a desired dose to be delivered by device 10. In some embodiments, rotating the dose button 30 in one direction relative to the housing 12 causes the dose button 30 to axially translate proximally relative to the housing 12, and rotating the dose button 30 in the opposite direction relative to the housing 12 causes the dose button 30 to axially translate distally relative to the housing. In some embodiments, clockwise rotation of the dose button moves the dose button 30 distally, and counter-clockwise rotation of the dose button moves the dose button proximally, or vice versa.

In some embodiments, rotating the dose button 30 to axially translate the dose button 30 in the proximal direction serves to increase the set dose, and rotating the dose button 30 to axially translate the dose button 30 in the distal direction serves to decrease the set dose. The dose button 30 is adjustable in pre-defined rotational increments corresponding to the minimum incremental increase or decrease of the set dose during the dose setting operation. The dose button may include a detent mechanism such that each rotational increment produces an audible and/or tactile "click." For example, one increment or "click" may equal one-half or one unit of medication.

In some embodiments, the set dose amount may be visible to the user via the dial indicator markings shown through a dosage window 36. During the dose setting mode, the actuator assembly, which includes the dose button 30 and clutch 52, moves axially and rotationally with the dose-setting assembly, which includes the flange 38 and the dose-setting screw 32.

Dose-setting screw 32 and flange 38 are fixed rotationally to one another, and rotate and move proximally during dose setting, due to the threaded connection of the dose-setting screw 32 with housing 12. During this dose setting motion, the dose button 30 is rotationally fixed relative to the flange 38 and the dose-setting screw 32 by complementary splines 74 of flange 38 and clutch 52 (FIG. 4), which are urged together by the bias member 68. In the course of dose setting, the dose-setting screw 32, flange 38, clutch 52, and dose button 30 together move relative to the housing 12 in a spiral manner (i.e. simultaneous rotation and axial translation) from a "start" position to an "end" position. This rotation and translation relative to the housing is in proportion to the amount of dose set by operation of the medication delivery device 10.

Once the desired dose is set, device 10 is manipulated so the injection needle 24 properly penetrates, for example, a user's skin. The dose dispensing mode of operation is initiated in response to an axial distal force applied to the proximal face 60 of dose button 30. The axial force is applied by the user directly to dose button 30. This causes axial movement of the actuator assembly (dose button 30 and clutch 52) in the distal direction relative to housing 12.

The axial shifting motion of the actuator assembly compresses biasing member 68 and reduces or closes the gap between dose button 30 and the tubular flange 38. This relative axial movement separates the complementary splines 74 on clutch 52 and flange 38, and thereby disengages the dose button 30 from being rotationally fixed to the flange 38 and the dose-setting screw 32. In particular, the dose-setting screw 32 is rotationally uncoupled from the dose button 30 to allow backdriving rotation of the dose-setting screw 32 relative to the dose button 30 and the housing 12. Also, while the dose-setting screw 32 and flange 38 are free to rotate relative to the housing 12, the dose button 30 is held from rotating relative to the housing 12 by the user's engagement of dose button 30 by pressing against it.

As dose button 30 and clutch 52 are continued to be axially plunged without rotation relative to housing 12, dose-setting screw 32 screws back into housing 12 as it spins relative to dose button 30. The dose markings that indicate the amount still remaining to be injected are visible through window 36. As dose-setting screw 32 screws down distally to advance sleeve 34, drive member 28 is advanced distally to push piston 26 through reservoir 20 and expel medication through needle 24.

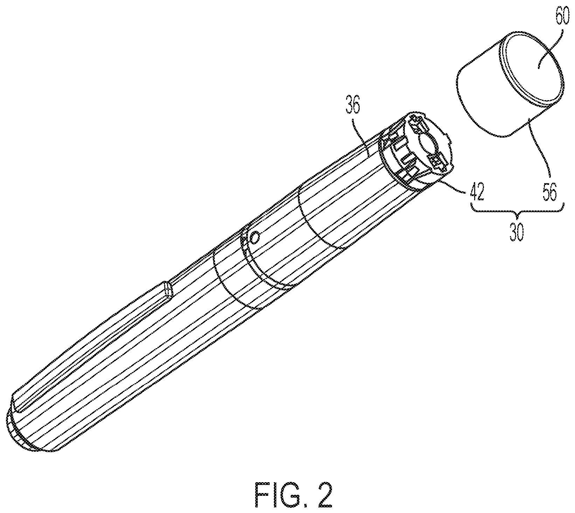

During the dose dispensing operation, the amount of medicine expelled from the medication delivery device is proportional to the amount of rotational movement of the dose-setting screw 32 relative to the housing 12 as the dose-setting screw 32 screws back into housing 12. In some embodiments, because the dose button 30 is rotationally fixed relative to the housing 12 during the dose dispensing mode, the amount of medicine expelled from the medication delivery device may be viewed as being proportional to the amount of rotational movement of the dose-setting screw 32 relative to the dose button 30 as the dose-setting screw 32 screws back into housing 12. The injection is completed when the internal threading of dose-setting screw 32 has reached the distal end of the corresponding outer threading of sleeve 34 (FIG. 4). Device 10 is then once again arranged in a ready state or zero dose position as shown in FIGS. 2 and 4.

As discussed above, the dose delivered may be derived based on the amount of rotation of the dose-setting assembly (flange 38 and dose-setting screw 32) relative to the actuator assembly (clutch 52 and dose button 30) during dose delivery. This rotation may be determined by detecting the incremental movements of the dose-setting assembly which are "counted" as the dose-setting assembly is rotated during dose delivery.

Further details of the design and operation of an exemplary delivery device 10 may be found in U.S. Patent No. 7,291,132, entitled Medication Dispensing Apparatus with Triple Screw Threads for Mechanical Advantage. Another example of the delivery device is an auto-injector device that may be found in U.S. Patent No. 8,734,394, entitled "Automatic Injection Device With Delay Mechanism Including Dual Functioning Biasing Member", where such device being modified with one or more various sensor systems described herein to determine an amount of medication delivered from the medication delivery device based on the sensing of relative rotation within the medication delivery device. Another example of the delivery device is a reusable pen device that may be found in U.S. Patent No. 7,195,616, entitled "Medication Injector Apparatus with Drive Assembly that Facilitates Reset", where such device being modified with one or more various sensor systems described herein to determine an amount of medication delivered from the medication delivery device based on the sensing of relative rotation within the medication delivery device.

Described herein is a dose detection system that may be operable to determine the amount of dose delivered based on relative rotation between a dose setting and/or delivery member and the device body. The dose setting and/or delivery member may include the flange 38, screw 32, or a combination of both as a monolithic unit. The dose detection system utilizes a dose setting member attached to the device body and rotatable relative to the device body about an axis of rotation during dose delivery. A sensed element is attached to and rotationally fixed with the dose setting member. An actuator is attached to the device body and is held against rotation relative to the device body during dose delivery. The sensed element thereby rotates relative to the actuator during dose delivery in relation to the amount of dose delivered.

In some embodiments, the dose detection system comprises a rotational sensor attached to the actuator assembly and a sensed element that includes surface features that are equally radially spaced about the axis of rotation of the sensed element.

In some embodiments, the dose detection systems may include a sensor and a sensed component attached to components of the medication delivery device. The term "attached" encompasses any manner of securing the position of a component to another component or to a member of the medication delivery device such that they are operable as described herein. For example, a sensor may be attached to a component of the medication delivery device by being directly positioned on, received within, integral with, or otherwise connected to, the component. Connections may include, for example, connections formed by frictional engagement, splines, a snap or press fit, sonic welding or adhesive.

The term "directly attached" is used to describe an attachment in which two components, or a component and a member, are physically secured together with no intermediate member, other than attachment components. An attachment component may comprise a fastener, adapter or other part of a fastening system, such as a compressible membrane interposed between the two components to facilitate the attachment. A "direct attachment" is distinguished from attachment where the components/members are coupled by one or more intermediate functional members.

The term "fixed" is used to denote that an indicated movement either can or cannot occur. For example, a first member is "fixed rotationally" with a second member if the two members are required to move together in rotation. In one aspect, a member may be "fixed" relative to another member functionally, rather than structurally. For example, a member may be pressed against another member such that the frictional engagement between the two members fixes them together rotationally, while the two members may not be fixed together absent the pressing of the first member.

Various sensor arrangements are contemplated herein. In general, the sensor arrangements comprise a sensor and a sensed component. The term "sensor" refers to any component which is able to detect the relative position or movement of the sensed component. The sensor may be used with associated electrical components to operate the sensor. The "sensed component" is any component for which the sensor is able to detect the position and/or movement of the sensed component relative to the sensor. For the dose detection system, the sensed component rotates relative to the sensor, which is able to detect the rotational movement of the sensed component. The sensor may comprise one or more sensing elements, and the sensed component may comprise one or more sensed elements. The sensor detects the movement of the sensed component and provides outputs representative of the movement of the sensed component.

Illustratively, the dose detection system includes an electronics assembly suitable for operation of the sensor arrangement as described herein. The medication delivery device may include a controller that is operably connected to the sensor to receive outputs from the sensor. The controller begins receiving generated signals from the sensor data indicative of counts from first to last one for a total number of counts that is used for determining total displacement, e.g. angular displacement. In the case of detecting an angular movement of a dose-setting assembly, the controller may be configured to receive data indicative of the angular movement of the dose-setting assembly that can be used to determine from the outputs the amount of dose delivered by operation of the medication delivery device. The controller may be configured to determine from the outputs the amount of dose delivered by operation of the medication delivery device. The controller may include conventional components such as a processor, power supply, memory, microcontrollers, etc. Alternatively, at least some components may be provided separately, such as by means of a computer, smart phone or other device. Means are then provided to operably connect the external controller components with the sensor at appropriate times, such as by a wired or wireless connection.

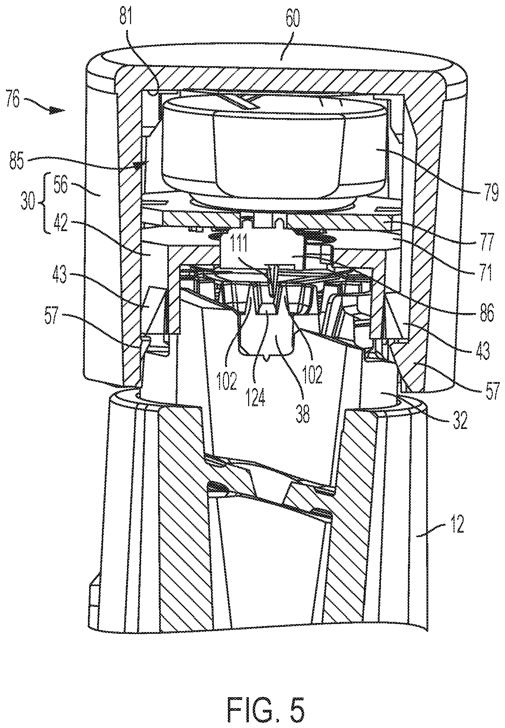

According to one aspect, the electronics assembly includes a sensor arrangement including one or more sensors operatively communicating with a processor for receiving signals from the sensor representative of the sensed rotation. An exemplary electronics assembly 76 is shown in FIGS. 5-7 and can include a sensor 86, and a printed circuit board (PCB) 77 having a plurality of electronic components. The printed circuit board may be a flexible printed circuit board. The circuit board of the electronics assembly 76 may include a microcontroller unit (MCU) as the controller comprising at least one processing core and internal memory. The electronics assembly may include a power source 79, e.g. a battery, illustratively a coin cell battery, for powering the components. The controller of electronics assembly 76 may include control logic operative to perform the operations described herein, including detecting the angular movement of the dose-setting assembly during dose setting and/or dose delivery and/or detecting a dose delivered by medication delivery device 10 based on a detected rotation of the dose-setting assembly relative to the actuator assembly. Many, if not all of the components of the electronics assembly, may be contained in a compartment 85 within the dose button 30. In some embodiments, the compartment 85 may be defined between a proximal surface 71 of support 42 of the dose button and a distal surface 81 of the cover 56 of the dose button. In the embodiment shown in FIG. 5, the electronics assembly 76 is permanently integrated within the dose button 30 of the delivery device. In other embodiments, the electronics assembly is provided as a module that can be removably attached to the actuator assembly of the medication delivery device.

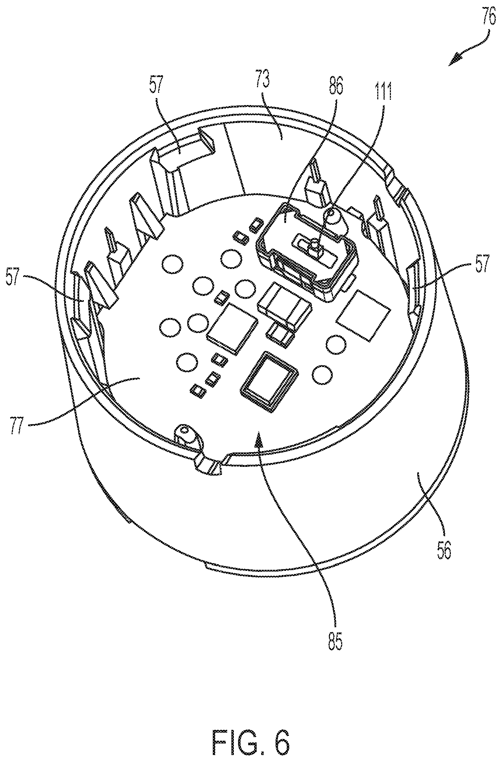

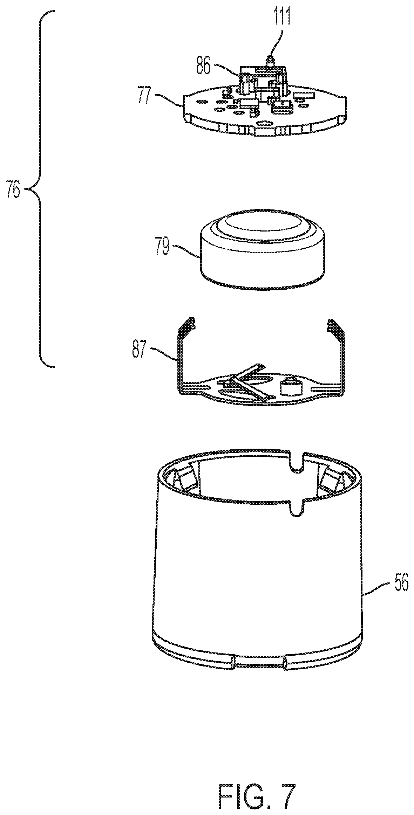

An underside view of the electronics assembly 76 held within the cover 56 is shown in FIG. 6, and an exploded view of the electronics assembly 76 is shown in FIG. 7. As shown in FIGS. 6 and 7, the electronics assembly 76 may include a printed circuit board (PCB) 77 and a sensor 86 having a contact surface 111. As shown in FIG. 7, the electronics assembly 76 may also include a battery 79 and a battery cage 87. Other sensors described herein, such as, for example, a magnetic sensor, may not include a contact surface but operate in a non-contact manner.

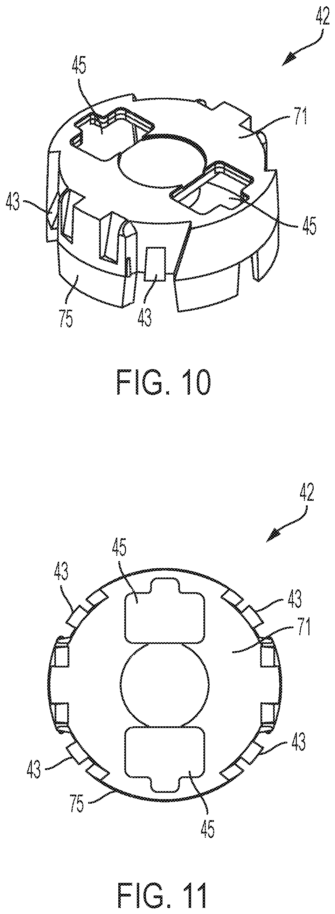

In some embodiments, at least a portion of the sensor 86 extends out of the compartment 85 of the dose button 30. As best seen in FIGS. 10 and 11, the support 42 of the dose button 30 may include one or more openings 45 through which the sensor 86 can extend through. In some embodiments, during assembly of the medication delivery device, the contact surface 111 of the sensor 86 is passed through the opening 45 of the support 42. This may permit the contact surface 111 of the sensor to interact with a component that is external to the compartment 85 of the dose button 30. In some embodiments, while only one of the openings 45 in the support 42 is needed to accommodate a sensor, a second opening may be provided, e.g. for symmetry of the support component, which help with manufacturing of the component and/or assembly of the component with the medication delivery device. When the sensor lacks a contact surface, the sensor may be positioned over the openings 45 without any sensor portion extending through the opening or with a portion extending through the opening but not configured to engage the sensed component.

The controller of electronics assembly 76 may be operative to store the total angular movement used for determining dose delivery and/or the detected dose delivery in local memory (e.g., internal flash memory or on-board EEPROM). The controller may be further operative to wirelessly transmit a signal representative of the total counts, total angular movement, and/or detected dose to an external device, such as a user's mobile device or a remote server. Transmission may, for example, be over a Bluetooth low energy (BLE) or other suitable short or long range wireless communication protocol. Illustratively, the BLE control logic and controller are integrated on the same circuit.

As discussed, according to one aspect, the dose detection system involves detecting relative rotational movement between two assemblies of the medication delivery device. With the extent of rotation having a known relationship to the amount of a delivered dose, the sensor operates to detect the amount of angular movement from the start of a dose injection to the end of the dose injection. For example, in some embodiments, the relationship for a pen injector is that an angular displacement of a dose-setting assembly of 18° is the equivalent of one unit of dose, although other angular relationships are also suitable, such as, for example, 9, 10, 15, 20, 24 or 36 degrees may be used for a unit or a half unit. The sensor system is operable to determine the total angular displacement of a dose setting member during dose delivery. Thus, if the angular displacement is 90°, then 5 units of dose have been delivered.

The angular displacement is determined by counting increments of dose amounts as the injection proceeds. For example, a sensing system may use a repeating pattern of a sensed element, such that each repetition is an indication of a predetermined degree of angular rotation. Conveniently, the pattern may be established such that each repetition corresponds to the minimum increment of dose that can be set with the medication delivery device.

The dose detection system components may be permanently or removably attached to the medication delivery device. In some embodiments, at least some of the dose detection system components are provided in the form of a module that is removably attached to the medication delivery device. In other embodiments, the dose detection system components are permanently attached to the medication delivery device.

In some embodiments, a sensor may detect, during dose delivery, the relative rotation of a sensed component that is rotationally fixed to the dose-setting screw 32, from which is determined the amount of a dose delivered by the medication delivery device. In an illustrative embodiment, a rotational sensor is attached, and rotationally fixed, to the actuator assembly. The actuator assembly does not rotate relative to the device housing during dose delivery.

In some embodiments, a sensed component is attached, and rotationally fixed, to the dose-setting screw 32, which rotates relative to the dose button 30 and the device housing 12 during dose delivery. In some of the embodiments described herein, the sensed component includes a ring structure having a plurality of proximally extending projections circumferentially disposed relative to one another. Projections are shaped and sized to deflect a movable element of the rotational sensor. One illustrative embodiment of such a sensed component is tubular flange 38, best seen in FIGS. 3, 5, 8, and 9. Embodiments described herein may be provided for a module that is removably attachable to the dose button of the delivery device or integrated within the dose button of the delivery device.

During dose delivery, dose-setting screw 32 is free to rotate relative to dose button 30. In the illustrative embodiment, the electronics assembly 76 is rotationally fixed with the dose button 30 and does not rotate during dose delivery.

As seen in FIGS. 2, 3 and 5, the dose button 30 comprises a cover 56 coupled to a support 42. An electronics assembly 76 may be at least partially contained within a compartment 85 defined between the cover 56 and the support. In some embodiments, the cover and support have corresponding splines that engage with one another to couple the cover and support together. For example, in some embodiments, the cover 56 may couple to the support 42 via one or more snaps 57 on the cover 56 and corresponding to one or more protrusions 43 on the support. As seen in FIG. 5 and 6, the snaps 57 on the cover 56 may be directed radially inwardly from an inner circumferential sidewall 73. As seen in FIGS. 5, 10 and 11, the protrusions 43 on the support 42 may be directed radially outwardly from an outer circumferential sidewall 75 of the support 42. The protrusions 43 may form a triangular ramp shape.

The snaps 57 on the cover 56 are configured to snap over and mate with the protrusions 43 on the support to couple the cover to the support. In some embodiments, the protrusion on the support comprises a continuous annular protrusion around the outer circumferential sidewall of the support. The cover 56 may attach to the support 42 via frictional engagement, interference fit or any other suitable fit. In some embodiments, the cover 56 is permanently fixed to the support 42 during assembly, e.g. via ultrasonic welding, adhesive, or other suitable fixation approach.

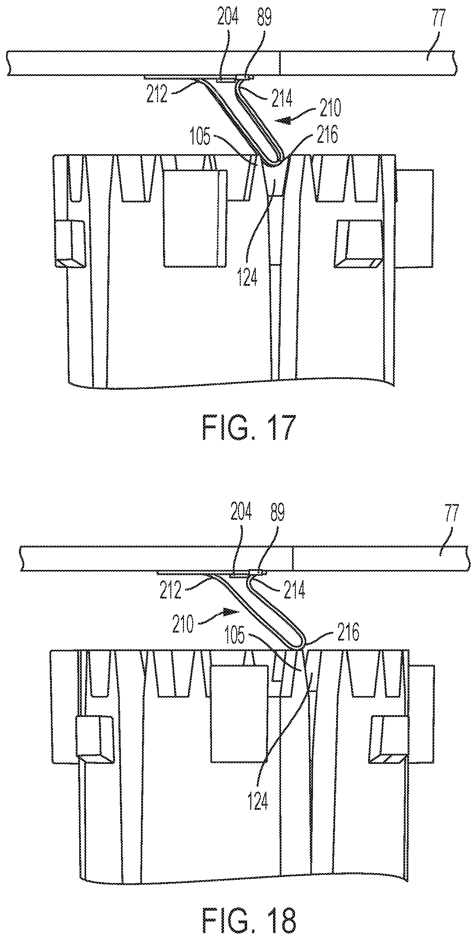

As seen in FIGS. 8 and 9, the tubular flange 38 may include a plurality of axially directed teeth 102 that may be equally radially spaced about a rotation axis and may be arranged to correlate to the equivalent of one or incremental unit of dose. In this illustrative embodiment, the tubular flange 38 includes 20 teeth 102 that are equally rotationally spaced from one another, such that the rotation distance between two adjacent teeth corresponds to 18 degrees of rotation. Thus, with the tubular flange 38 of FIG. 8, 18 degrees of rotation of the tubular flange 38 may be used to represent one dosage unit or a half dosage unit. It should be appreciated that, in other embodiments, different total numbers of teeth may be used to create other angular relationships, such as, for example, 9, 10, 15, 18, 20, 24 or 36 degrees may be used for a unit or 0.5 unit.

A recess 124 may be defined between each pair of adjacent teeth 102. Each tooth 102 may have an approximately triangular shaped profile, each having a surface 120 against which a contact surface 111 of a sensor may slide.

In some embodiments, the sensor for detecting rotation of the tubular flange includes a movable element that has a contact portion capable of resting against the teeth of the tubular flange and is spring-biased such that the contact surface is configured to slide against and over the teeth during rotation of the flange relative to the actuator assembly during dose delivery. The sensor is responsive to the movement of the contact portion over the teeth and generates signals corresponding to the flange. A controller is responsive to the signals generated by the sensor to determine a dose count for determining the dosage delivered based on the detected rotation of the flange relative to the actuator assembly during dose delivery.

The contact surface may be biased against the physical features of the tubular flange to ensure proper contact between the contact surface and the physical features during rotation. In one embodiment, the movable element is a resilient member having one portion attached to the actuator at a location displaced from the contact surface. In one example, the movable element is a following member comprising a beam attached at one end to the actuator and having the contact surface at the other end. The beam is flexed to urge the contact surface in the direction of the surface features. Alternatively, the movable element may be biased in any of a variety of other ways. In addition to the use of a resilient beam, the biasing may be provided, for example, by use of a spring component. Such spring component may for example comprise a compression, tension, or torsion coil spring. In yet other embodiments, the movable element may be biased against the surface features of the sensed element by a separate resilient member or spring component bearing against the movable element.

FIG. 5 depicts an illustrative embodiment of a sensor 86 having a contact surface 111 interacting with teeth 102 of a tubular flange 38. As the flange 38 rotates relative to the dose button 30 during delivery, the teeth 102 of the flange contact and slide against the contact surface 111 of the sensor 86, causing the contact surface 111 to move in an oscillating manner. The movement of the contact surface 111 may be a combination of axial and lateral movement as the contact surface 111 slides into and out of the recesses 124 defined between the teeth 102 of the flange 38. The sensor 86 may be configured to track the movement of the contact surface 111 and associate the movement with an output signal that is sent to a controller.

As alternative to teeth on the tubular flange, surface features that interact with the sensor may comprise anything detectable by the sensor. The sensor arrangement may be based on a variety of sensed characteristics, including tactile, optical, electrical and magnetic properties, for example. In the illustrative embodiments shown in the figures, the surface features are physical features which allow for detection of incremental movements as the dose-setting assembly rotates relative to the actuator assembly. In alternative embodiments, the sensor may be a piezoelectric sensor, a magnetic sensor such as a Hall effect sensor, where the teeth have magnets or distinguishable magnetic properties, a capacitive or inductive sensor with the teeth have metallic properties, an accelerometer for detecting vibration, e.g. of a ratcheting or other detent mechanism, where vibration can be correlated with rotational movement, an optical sensor such as a reflective sensor, an interrupter sensor, or an optical encoder, or any other sensor suitable for sensing rotation of a first component relative to a second component.

In some embodiments, when a user presses axially on face 60 of the dose button 30, the dose button 30 advances distally relative to the housing 12, compressing spring 68. Continued pressing of the dose button 30 distally results in back driving of the dose-setting screw 32 in a spiral direction relative to housing 12. As a result, the dose-setting screw 32 and flange 38 are driven to rotate by the axially pressing upon the dose button 30. In some embodiments, the dose detection system is operable for dose detection only while the dose button is being pressed.

In some embodiments, the electronics assembly may include a clock or timer to determine the time elapsed between counts caused by trigger of the rotational sensor from the surface features of the sensed element. When no counts have been detected by the controller after a period of time this may be used to indicate that the dose has completed.

In some embodiments, a single sensing system may be employed for both dose detection sensing and wake-up activation. For example, upon the initial sensing of rotation of the sensed element by the sensor, the controller is configured to allow wake-up or activation of the electronics assembly to a greater or full power state. The wake-up feature is configured to allow power transmission from the power source (shown as battery) for powering up the electronic components for dose sensing in order to minimize inadvertent power loss or usage when a dose dispensing event is not occurring. In other embodiments, a separate wake-up switch may be provided and arranged within the dose button housing and triggered when the dose button is in its distal position. After activation of the electronics assembly, the controller begins receiving generated signals from the rotational sensor indicative of counts from first to last one for a total number of counts that is used for determining total angular displacement and thus the amount of dose delivered.

In some embodiments, the electronics assembly may have a controller that is configured to receive an output signal from a rotational sensor. The controller of the electronics assembly may be programmed to convert the intermediate signal to a conditioned digital signal, which may be a single step/square wave with a predetermined width representing a predetermined time. In some embodiments, output signals that are less than a predetermined level may be filtered out and ignored.

According to one aspect, a medication delivery device includes a repeatedly activatable switch that may serve as a sensor. In some embodiments, the switch serves as the rotational sensor in the dose detection system described above. In other embodiments, however, the switch may be used to detect other activity such as removal of a cap.

In some embodiments, the switch comprises a conductive pad and a cantilevered arm that is moveable relative to the conductive pad. The cantilevered arm may be mounted to the PCB at a first end, and a second end of the arm may be unattached and free to move relative to the printed circuit board. In one embodiment, the conductive pad includes an axially facing surface coplanar with the PCB that is in confronting relationship with the axially moving arm. In another embodiment, the conductive pad includes a radially facing surface transverse to the PCB to engage the radially moving arm after its initial axial engagement with the PCB.

According to one aspect, the switch may have one or more features that help to permit the switch to repeatedly change its state between first and second states, such as, for example, open and close. The switch may have one or more features that help the switch to avoid plastic deformation during repeated opening and closing, thus helping to maintain durability of the switch.

In some embodiments, at the first end of the cantilevered arm, where the arm attaches to the printed circuit board, the arm has a first curved portion. During switch closure, this first curved portion may be moved toward a straight configuration, and during switch opening, the straight configuration may be moved back toward the curved configuration. In the unstressed state, this first portion may be biased toward the curved configuration. Accordingly, the cantilevered arm may act as a spring that stores potential energy as it is moved during sliding interaction against a sensed component, where the stored potential energy is released to move the arm back toward the unstressed state when the sliding contact force against the arm has decreased.

In some embodiments, the cantilevered arm may transition from the first curved portion to a second curved portion that is configured to move toward and contact the conductive pad that is mounted to the PCB. Contact between the second curved portion and the conductive pad closes or opens the switch, while lack of contact between the second curved portion and the conductive pad opens or closes the switch.

In some embodiments, the cantilevered arm may include a third curved portion configured to contact and slide against the sensed component, e.g. against the teeth of a rotating tubular flange 38 shown in FIGS. 8 and 9. In some embodiments, the third curved portion connects the first curved portion to the second curved portion. In such embodiments, the second curved portion, which is configured to contact the conductive pad, may be located at the second end of the cantilevered arm, i.e. where the cantilevered arm truncates. In other embodiments, the second curved portion connects the first and third curved portions. In such embodiments, the third curved portion, which is configured to contact the sensed component, may be located at the second end of the cantilevered arm, i.e. where the cantilevered arm truncates.

In some embodiments, having the curved portions of the cantilevered arm may help to avoid high strain concentrations in the arm, and may thus help to prevent plastic deformation of the arm. However, it should be appreciated that one or more of the curved portions may be shaped differently in other embodiments.

In some embodiments, the switch may have one or more features that help the switch to provide a cleaner, more easily readable output signal such that a controller can more accurately identify when the switch is open and when the switch is closed. In some embodiments, a blocking protrusion may be provided to interact with the second curved portion of the cantilevered arm as the arm makes contact with the conductive pad. In another embodiment, the blocking protrusion is the conductive pad, such as, for example, including a radially facing surface with conductive properties, to interact with the second curved portion of the cantilevered arm as the arm makes contact with a zone of the PCB. This blocking protrusion may be situated directly adjacent to or near the conductive pad and may prevent the arm from moving past the conductive pad. In some embodiments, the presence of the blocking protrusion may help to reduce "bounce" of the cantilevered arm that may aid in producing a cleaner output signal from the switch. In some cases, "bounce" from the cantilevered arm may cause the arm to rapidly and repeatedly contact and separate from the conductive pad in a short period of time, which can create a noisy output signal that may be difficult for a controller to interpret. The blocking protrusion may help to provide sustained contact between the cantilevered arm and the conductive pad to provide a cleaner output signal. In some embodiments, the blocking protrusion may be made from a shock-absorbing material that may help to deaden the impact of the cantilevered arm against the blocking protrusion, in order to decrease bounce or other vibration.

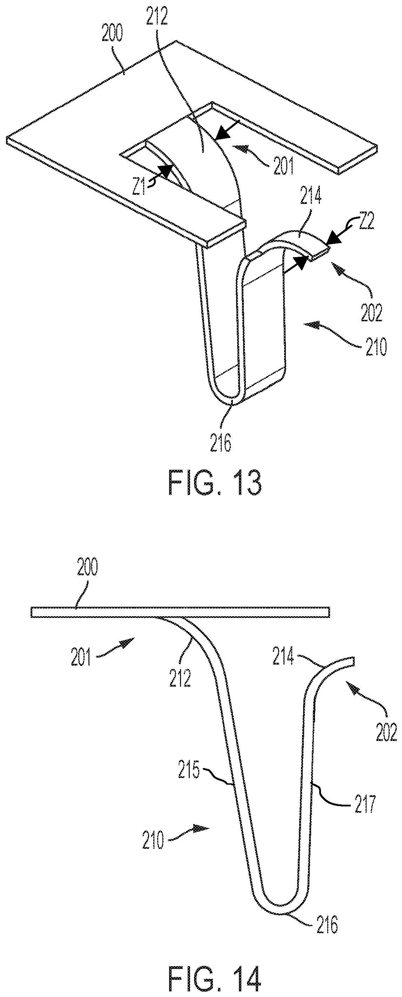

In some embodiments, the switch may include a single, monolithic metal component. The metal component may be formed via stamping, die casting, hydroforming, or any other suitable manufacturing method. The metal component may have various different shapes. Examples of illustrative embodiments of various metal components are shown in FIGS. 13, 20, 22, 26 and 29, and will be discussed in more detail below.

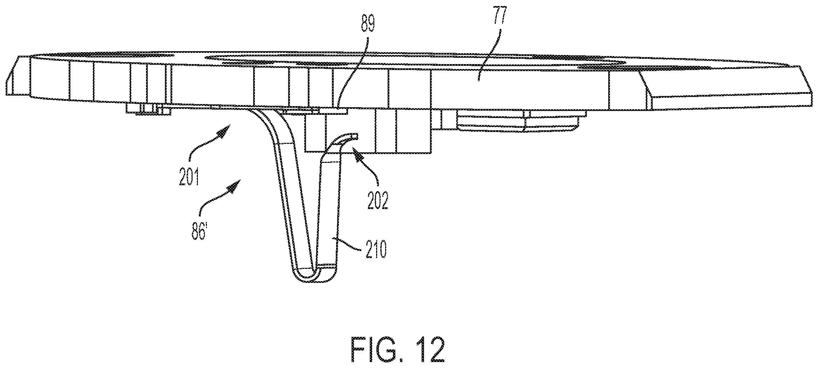

One illustrative example of a switch is shown in FIGS. 12-14, which depicts the sensor 86' as a switch having a conductive pad 89 and a cantilevered arm 210. The conductive pad 89 and a first end 201 of the cantilevered arm 210 are mounted to a PCB 77. The conductive pad 89 may be coplanar with the PCB. The conductive pad may also be associated with another element and/or out of plane with the PCB.

As best seen in FIGS. 13 and 14, the cantilevered arm 210 begins at a first curved portion 212 at its first end 201 and truncates at a second curved portion 214 at its second end 202. The arm also includes a U-shaped third curved portion 216 that connects the first curved portion 212 to the second curved portion 214. Arm 210 may also include linear portions interconnecting the respective curved portions 212, 214, 216. The length of the linear portions may vary and may be different from what is shown in the figures. For example, a first linear portion 215 is coupled between the first and third curved portions 212, 216, and a second linear portion 217 is coupled between the second and third curved portions 214, 216, as shown in FIG. 14. The second curved portion 214, shown here curved in a manner to position the second end 202 to face away from the first end 201 and/or first curved portion, is configured to contact with a conductive pad, and the third curved portion 216 is configured to contact with a sensed component such as the rotating tubular flange 38 shown in FIGS. 8 and 9. The use of the term "curved" also encompasses other transitions between two surfaces such as angular or V-shaped.

The switch also includes a base 200 that is connected to the cantilevered arm 210. The base 200 is connected to the PCB to connect cantilevered arm to the PCB. The base and the arm together may form a single monolithic component. In some embodiments, the base of any of the switch embodiments may be coupled to another portion of the device other than the PCB in a manner such that the functional movement of making selective contact with the conductive pad and the teeth. As shown in FIG. 13, the relative width Z2 of the second curved portion 214 is less than a width Z1 of the first curved portion 212.

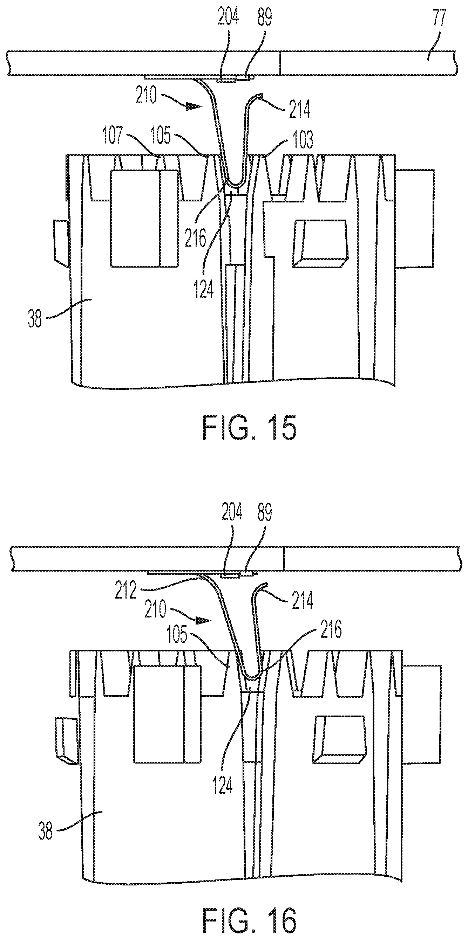

FIGS. 15-19 depict the cantilevered arm 210 of the switch interacting with the rotating flange 38 from FIGS. 8 and 9. FIG. 15 shows the arm 210 in an unstressed state, as the third curved portion 216 is situated within a recess 124 between two adjacent teeth 103, 105. The switch may be positioned in this state when the flange 38 is at its home or zero dose position, e.g. prior to use of the device, prior to setting a dosage, or after a dispense has completed and the device is ready for a dosage to be set.

In FIG. 16, the flange 38 has begun to rotate relative to the switch and the PCB 77. As a result, tooth 105 slides and pushes against the third curved portion 216 of the arm 210, causing the arm 210 to begin to deflect toward the proximal direction out of the recess 124. The first curved portion 212 begins to move toward a straightened configuration, and the second curved portion 214 begins to move toward the conductive pad 89. It is noted that all of the embodiments described herein in an unstressed state is disposed substantially axial along the axis, that is, the angle between the base and the arm is about 60-120 degrees, and when engaged by the teeth and moved to the stressed state, is moved toward the straightened configuration, that is, the angle between the base and the arm being now between 120-180 degrees (as shown, for example, in FIG. 17).

In FIG. 17, the flange 38 has rotated further than in FIG. 16, causing the tooth 105 to slide against and push the third curved portion 216 nearly completely out of the recess 124. The first curved portion 212 has moved even more toward a straightened configuration. As a result, the second curved portion 214 has made contact with the conductive pad 89, thereby closing the switch. The second curved portion also pressed against a blocking protrusion 204, which prevents the second curved portion from moving further toward the first curved portion 212, and may help to prevent the second curved portion from bouncing repeatedly against the conductive pad 89 in a rapid manner that may give rise to a noisy output signal. In the alternative, the second curved portion 214 may make contact with an axial zone of the PCB (where pad 89 is located as shown in FIG. 17). The second curved portion 214 also can move radially to press against a conductive radial surface (now functioning as the conductive component) of the blocking protrusion 204. The radial configuration can prevent the second curved portion 214 from moving closer toward the first curved portion 212, and may help to prevent the second curved portion from bouncing repeatedly against the zone in a rapid manner that may give rise to a noisy output signal.

In FIG. 18, the flange 38 has rotated further than in FIG. 17, and the third curved portion 216 has exited the recess 124 and is sliding across the top of tooth 105. The second curved portion 214 remains in contact with both the conductive pad 89 and/or the blocking protrusion 204, depending on the embodiment. Blocking protrusion 204 has prevented the second curved portion 214 from moving radially closer to the first curved portion 212.

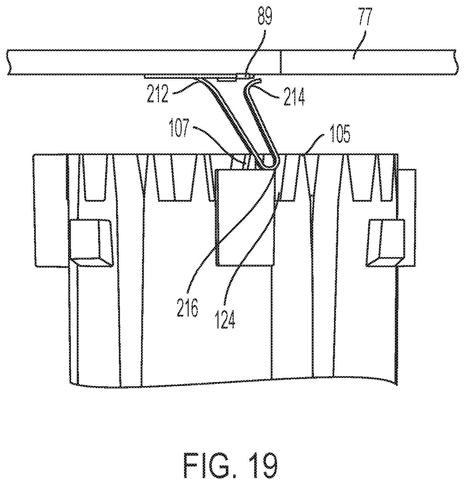

Finally, in FIG. 19, the flange 38 has rotated further than in FIG. 18, and the third curved portion 216 has stopped contacting tooth 105 and has now begun contacting the next adjacent tooth, 107. During this transition as the next tooth 107 is just beginning to push upon the arm 210, the arm, which is spring biased toward the position shown in FIG. 15, has swung back toward its unstressed state, thus causing the first curved portion 212 to move toward a more curved shape, resulting in movement of the third curved portion 216 toward a direction opposite to the rotation direction of the flange 38 and resulting in movement of the second curved portion 214 away from the conductive pad 89, thereby opening the switch. As the flange 38 rotates further, the cycle continues and the arm moves back toward the conductive pad to close the switch, and so on.

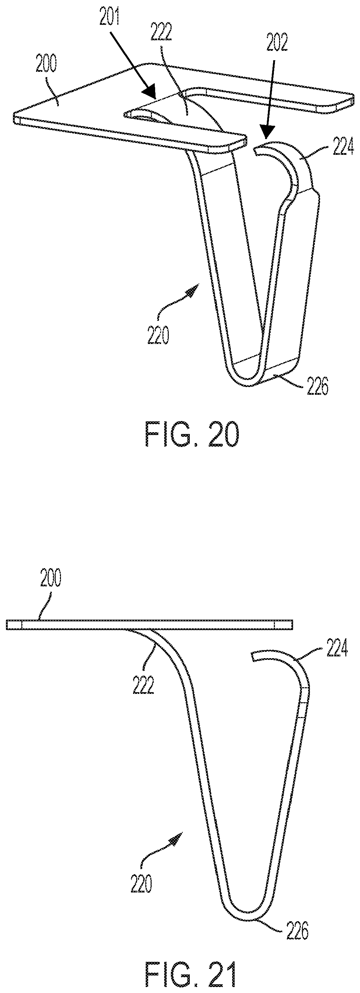

A first alternative embodiment of a switch is shown in FIGS. 20 and 21. This embodiment is similar to the embodiment shown in FIGS. 12-14, except that the second curved portion 224 is curved in the opposite direction than shown in FIG. 12, that is, to position the second end 202 facing toward the first end 201 and/or the first curved portion. In the embodiment of FIGS. 12-14, the second curved portion 214 is curved such that, when the cantilevered arm is in an unstressed state, a curvature of the second curved portion faces away from the first curved portion 212. In the embodiment of FIGS. 20-21, the second curved portion 224 is curved such that, when the cantilevered arm is in an unstressed state, a curvature of the second curved portion faces toward the first curved portion 222. Otherwise, the base 200, first curved portion 222, and third curved portion 226 of the embodiment of FIGS. 20-21 are the same as those of the embodiment of FIGS. 12-14. As shown in FIG. 20, the relative width of the second curved portion 224 is less than a width of the first curved portion 222, similar to the embodiment in FIG. 13.

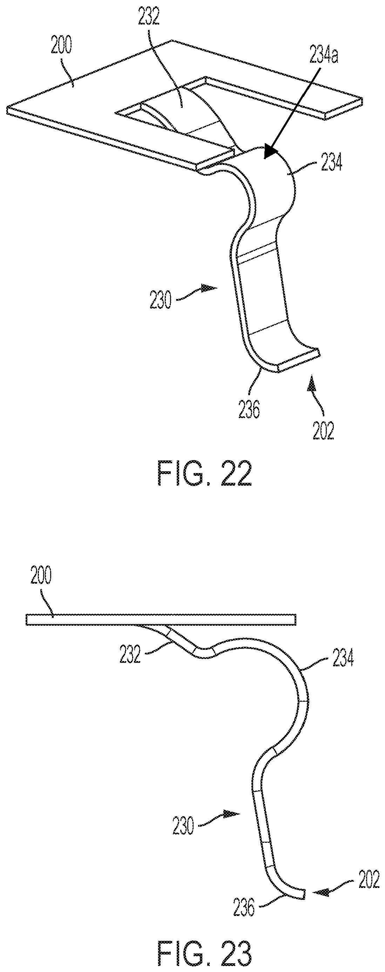

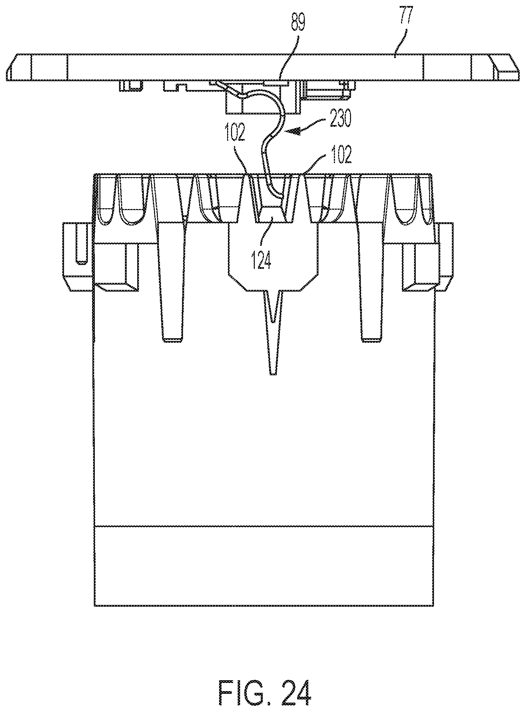

A second alternative embodiment of a switch is shown in FIGS. 22-23. In this embodiment, the first curved portion 232 is similar to the first curved portions of the other two embodiments, but the second the third curved portions are different. In this embodiment, the cantilevered arm 230 truncates at the second end 202 with the third curved portion 236 that is contactable with the teeth 102 instead of the second curved portion 234 shown in the prior embodiments. The third curved portion 236 is still configured to contact and slide against a sensed component such as the tubular flange, and the second curved portion 234 is still configured to contact with a conductive pad to close the switch. As a result, in this embodiment, the second curved portion 234 is coupled between the first curved portion 232 to the third curved portion 236, where linear portions are shown connecting the curved portions. Furthermore, the second curved portion 234 is U-shaped, where the bottom 234a of the U-shape has a curvature to face toward the PCB and to facilitate contact with the conductive pad 89, with reference to FIG. 24. To give this differently-shaped arm context, FIG. 24 shows the arm 230 interacting with teeth 102 of a rotating tubular flange 38, and interacting with a conductive pad 89.

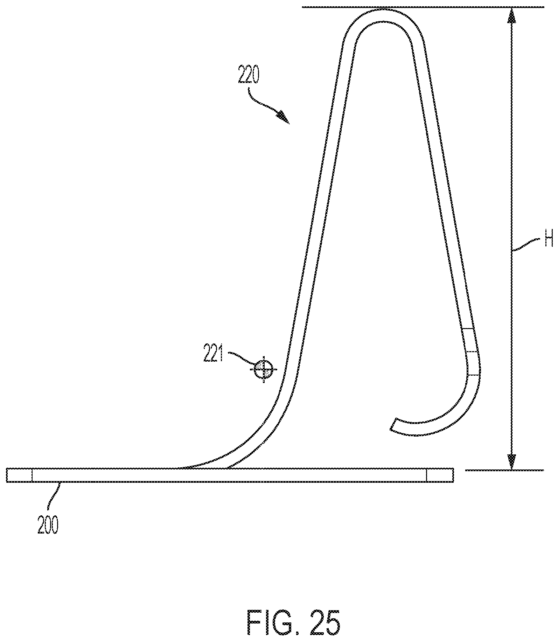

According to one aspect, the cantilevered arm may be shaped such that the center of mass of the arm is positioned over the base that is connected to the arm. For example, as shown in FIG. 25, in which the base and arm construct has been flipped upside-down, the center of mass 221 of the arm 220 is located over the base 200. Such a feature may aid in assembly of the base and arm with the PCB. For example, in some circumstances, it may be beneficial for a component to be able to stand up on its own, e.g. to utilize reflow soldering. Having the center of mass 221 of the arm 220 located over the base 200 permits the arm and base construct to stand up on its own on the PCB. It may also be beneficial for the center of mass to be relatively low, e.g. closer to the base than to the furthest point of the arm at height H. The embodiments of FIGS. 12-14 and FIGS. 22-23 are also examples of an arm shape having a center of mass that is positioned over the base.

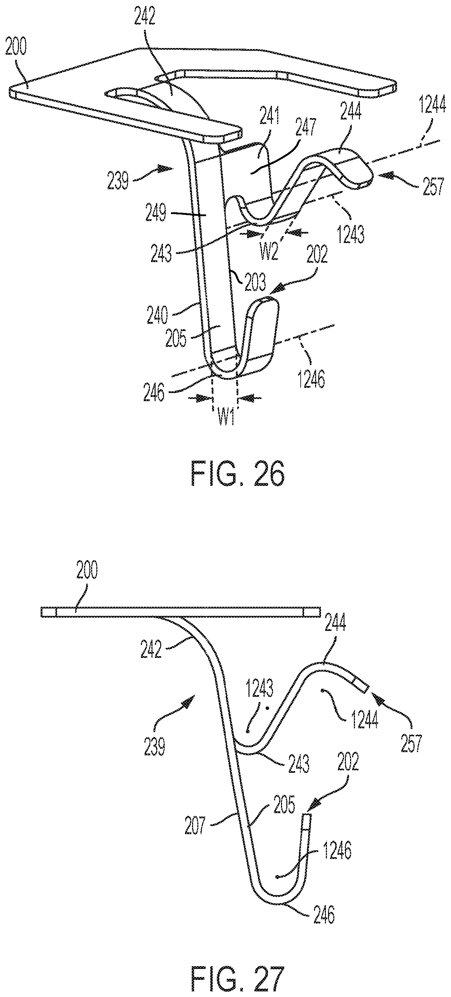

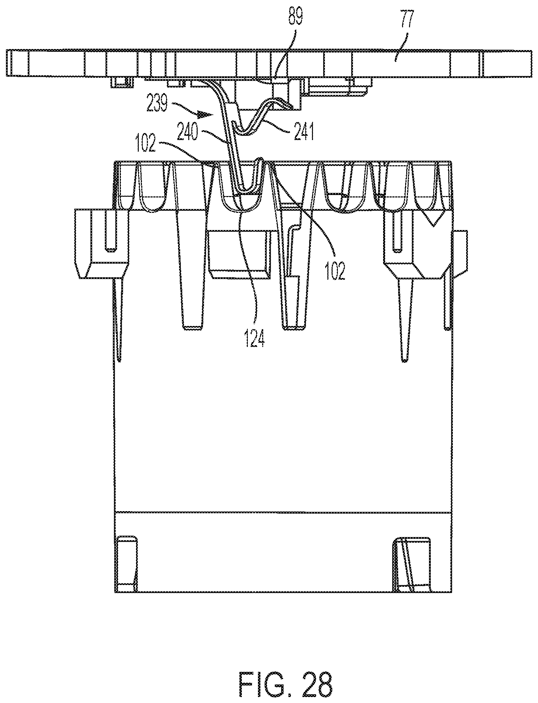

A third alternative embodiment of a switch is shown in FIGS. 26-28. In this embodiment, the switch includes a cantilevered arm 239 having a first branch 240 and a second branch 241, where one of the branches contacts the conductive pad and the other of the branches contacts the teeth 102. As seen in FIG. 28, for example, the second branch 241 is configured to contact conductive pad 89 to close the switch. The second branch 241 may be configured with the first branch 240 such that movement of the first branch 240 causes movement of the second branch 241. The first and second branches may be a monolithic unit or may be discrete components that are attached together such as by, for example, welding.

The second branch 241 may extend in a manner to be in circumferential alignment with the first branch 240, that is, such that the second branch overlaps the first branch 240. In other embodiments, the second branch 241 may be radially offset (inward as shown or outward) from the first branch 240. In some embodiments, the second branch 241 may be extended from a side face 203 of the first branch 240. However, in other embodiments, the second branch 241 may extend from another part of the first branch 240, such as, for example, a front face 205 or a rear face 207 of the first branch 239 or the side face opposite the side face 203. The cantilevered arm 239 includes a curved portion 242. Curved portion 242 is shown to be proximal to the branching location where the second branch 241 is coupled to the first branch 240. First branch 240 is shown having a linear portion 249 extending distally away from curved portion 242, and truncates at second end 202 with the curved portion 246 so that the end 202 faces distally toward the base 200. The curved portion 246 is configured to contact and slide against a sensed component such as the tubular flange. The second branch 241 includes a body portion 247 coupled to the side face 203, shown adjacent and coplanar with the linear portion 249 of the first branch 240, a first curved portion 243 for transitioning from distal to proximal directions, and a second curved portion 244 for transitioning back to the distal direction, where the first curved portion 243 is disposed between the body portion 247 and the second curved portions 244. Second branch 241 is terminated at an end 257, where end 257 is shown facing away from first branch 240. The first curved portion 243 is curved about axis 1243, and the second curved portion 244 is curved about axis 1244. In this embodiment, the axes 1243, 1244 about which the first and second curved portions 243, 244 are curved are parallel to one another. In some embodiments, the second branch 241 may form an undulating shape. The second curved portion 244 of the second branch 241 is configured to contact the conductive pad to close the switch, which due to the radial offset of the second branch 241 and second curved portion 244 from the first branch 240 to allow for the radial inward placement of the contact pad relative to the teeth 102. The first curved portion 243 of the second branch 241 may be U-shaped.

The curved portion 246 of the first branch is curved about axis 1246. In some embodiments, the axis 1246 of the curved portion 246 of the first branch 240 is parallel to the axis 1244 of the curved portion 244 of the second branch 241. The axes 1246, 1244, 1243 may be oriented generally orthogonal to the longitudinal axis AA.

FIG. 28 shows the first branch 240 positioned between two teeth 102 of a rotating tubular flange 38. As the tubular flange 38 rotates, the teeth 102 push up against the first branch 240, moving the first branch 240 proximally toward the PCB 77. Because the second branch 241 is extended from the first branch 240, proximal movement of the first branch 240 causes the second branch 241 to move correspondingly proximal toward and contact the conductive pad 89 on the PCB 77 to close the switch. As the first branch 240 deflects distally downward into a recess 124, the second branch 241 moves correspondingly distal with the first branch 240 and out of contact with the conductive pad 89 to open the switch.

The first branch 240 has a width W1 and the second branch 241 has a width W2. In the embodiment of FIG. 26, the widths W1 and W2 are the same. In other embodiments, however, the widths W1 and W2 may be different. In some embodiments, the width W1 of the first branch 240 may be larger than the width W2 of the second branch 241, or vice versa.

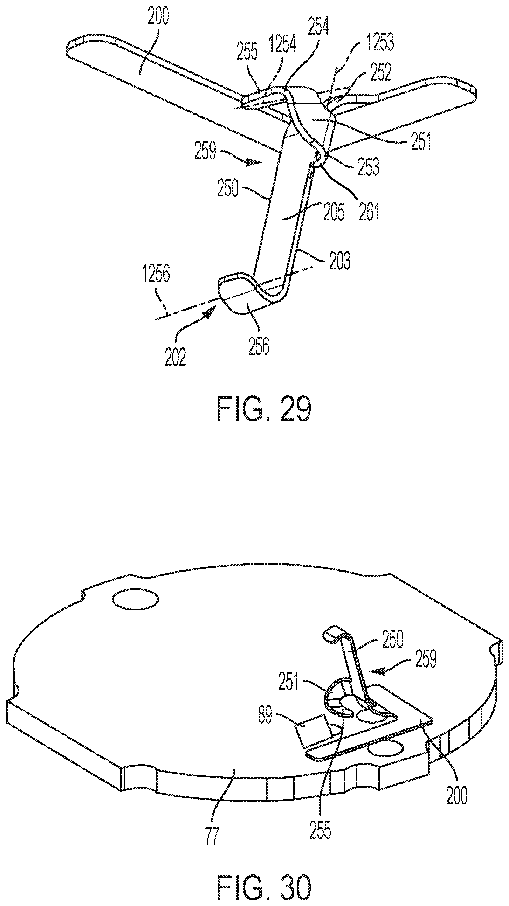

A fourth alternative embodiment of a switch is shown in FIGS. 29 and 30. In this embodiment, the switch includes a cantilevered arm 259 having a first branch 250 and a second branch 251. In this embodiment, the second branch 251 is shaped differently from that of the embodiment of FIG. 26. The second branch 251 includes a body portion 261 (shown coupled to the side face 203; however, can be coupled along another face of the first branch), a first curved portion 253 for transitioning from the inner radial direction to the circumferential direction, and a second curved portion 254 for transitioning to the outer radial direction. The first curved portion 253 is curved about axis 1253, and the second curved portion 254 is curved about axis 1254. Second branch 251 is terminated at an end 255, where end 255 is faces radially away from the longitudinal axis AA. In this embodiment, the axis 1253 of the first curved portion 253 and the axis 1254 of the second curved portion 254 are not parallel to one another. As seen in FIG. 30, the second branch 251 forms a C-shape that bends out of plane.

The curved portion 256 of the first branch is curved about axis 1256. In this embodiment, the axis 1256 of the curved portion 256 of the first branch 250 is not parallel to the axis 1254 of the curved portion 254 of the second branch 251. Axis 1256 is generally orthogonal to the longitudinal axis AA and axis 1253. Axis 1253 generally runs along the longitudinal axis AA, while axis 1254 is transverse to both axes 1256, 1253.

The second branch 251 is configured to contact with a conductive pad to close the switch. In some embodiments, the second branch extends from side face 203. However, in other embodiments, the second branch may extend from front face 205 or a rear face. The curved portion 256 of the first branch 250 is configured to contact and slide against a sensed component such as the tubular flange. The distal end 255 of the second branch is configured to contact the conductive pad to close the switch. Here, the end 255 is positioned to be in alignment with and/or overlapping the curved portion 256 to allow for the conductive pad 89 to be placed axially overlapping the teeth 102. In another embodiment, the conductive pad 89 may be radially inward from base 200 in a position for selective contact with the curved portion 254 rather than the end 255. The conductive pad 89 may be out of plane from the PCB 77 such as shown in FIG. 30 in an angled position in order to facilitate selective contacting with the curved portion 254 during sensing.

In some embodiments, such as the embodiments of FIGS. 26 and 29, the switch includes a cantilevered arm having first and second branches that form one monolithic component. For example, the first and second branches may be stamped from one sheet of metal, or integrally formed via a die casting process. The first and second branches may be made of the same material, may have the same thickness, may have the same width, or any combination thereof. In other embodiments, however, the first and second branches may be made of different material, may have different thicknesses, may have different widths, or any combination thereof. The first and second arms may be differently shaped and/or have different material properties from one another.

According to one aspect, a user may receive digital feedback regarding how much medication remains within the medication delivery device. When the medication level of the medication delivery device becomes low, the user may receive an alert from the medication delivery device and/or from an external device informing the user of the low medication level. For example, the user may receive the alert from a mobile device, e.g. from an app, text message or SMS. An alert may be visual, auditory, tactile (e.g. a vibration) or any combination thereof.

In some embodiments, the user may be advised to refill their medication and/or, for re-useable medication delivery device, replace the medication cartridge with a new cartridge. In some embodiments, a refill request may be automatically sent to a pharmacy when a medication level is detected to be low.

The method by which medication level is determined may be accomplished in different ways. In some embodiments, an external device stores a record of the level of medication remaining in a medication delivery device. The medication delivery device may send communications to the external device, informing the external device of the dosage that was delivered from each medication delivery event. The medication delivery device may determine such dosage information from the sensor described above. The external device may then calculate and store the amount of medication remaining after each medication delivery event. For example, the external device may subtract the delivered dosage amount from the last-known remaining amount of medication.

In some embodiments, the information regarding the amount of medication remaining in a medication delivery device may be calculated and/or stored by the medication delivery device itself. The medication delivery device may then communicate to an external device how much medication remains in the medication delivery device.

In some embodiments, tracking the amount of medication remaining in a medication delivery device may be used for patient adherence purposes. A user, caretaker, healthcare provider, insurance payer, and/or a company creating the medication may wish to monitor whether the user is taking the medication at the prescribed amounts and/or times. In some embodiments, such information may be used in conjunction with other devices to improve treatment for the patient. For example, the medication delivery device may be used in conjunction with a glucose meter. Dosages delivered to a patient may be paired with glucose level information to determine information such as efficacy of the medication, efficacy of the patient's regimen, etc. Such information may help to improve patient treatment, e.g. by suggesting possible ways to improve the patient's regimen.

According to one aspect, a medication delivery device may have the ability to assist a user with finding the location of the medication delivery device. The inventors have appreciated that a user may, at times, have trouble finding their medication delivery device, particularly if it is portable and can be used in different locations. The inventors have recognized the need for a device location assist feature to help the user locate the device.

In some embodiments, the location of a medication delivery device is tracked by one or more mobile devices. For example, a medication delivery device may be configured to communicate with one or more mobile devices or other external devices such as a remote server. The communication may be one-way communication or two-way communication.

In some embodiments, the medication delivery device periodically advertises information such as a unique identifier. A mobile device may periodically scan for medication delivery devices, and if the advertising medication delivery device is in communication range with the mobile device, the mobile device would receive the communication from the medication delivery device. The mobile device, which may have a built-in GPS or other location-identifying ability, may then associate a location with each received communication. Particularly if the communication protocol between the medication delivery device and the mobile device is a short distance communication protocol, such as Bluetooth, the mobile device may assign the mobile device's own present location, or a radius around the mobile device's own present location, as the location of the medication delivery device. In some embodiments, when the mobile device no longer receives communications from the medication delivery device, indicating that perhaps the medication delivery device has been moved out of communication range from the mobile device, the mobile device stores a last-known location of the medication delivery device, which may be when the mobile device last received communication from the medication delivery device. This last-known location may be presented to a user to help the user determine the location of the medication delivery device.

In some embodiments, when a mobile device is brought into communication range with a medication delivery device, the mobile device may alert a user that a medication delivery device is nearby. This may be used to help the user physically find the medication delivery device.

In some embodiments, when a mobile device senses that a medication delivery device is no longer in communication range with the mobile device (e.g. the mobile device does not receive an advertisement from the medication delivery device within an expected time period), the mobile device may alert the user the medication delivery device is no longer nearby, or at least no longer close to the mobile device itself. Such a feature may help the user to avoid forgetting to bring the medication delivery device when the user leaves a location.

In some embodiments, multiple mobile devices may cooperate to help locate a medication delivery device. For example, a group of mobile devices may be configured to scan periodically for medication delivery devices. When one of the mobile devices locates a medication delivery device (e.g. by sensing that the medication delivery device is in communication range), the mobile device may communicate the identity and/or location of the found medication delivery device to the rest of the mobile devices. This may be used, for example, in a household setting where members of the household each have their own mobile device.

In some embodiments, the medication delivery device may include a built-in speaker. To help a user find the medication delivery device, the speaker may be triggered by the user to emit a sound. In some embodiments, a user may use a mobile device to trigger the speaker to emit a sound.

In some embodiments, the medication delivery device itself may have a built-in GPS or other location-identifying ability. The medication delivery device may communicate its location to a mobile device or other external device, such as directly to a remote server.

According to one aspect, the date, and in some embodiments, time, at which a medication delivery device is used for the first time is tracked.

One example use case for such a feature is determining medication expiration. For example, in some embodiments, a medication delivery device may communicate to an external device that user has opened, turned on, or otherwise activated the medication delivery device for the first time. The external device may check whether the medication has expired, by, for example, looking up an identification number of the medication delivery device in an expiration date database.

Another example use case for such a feature is to assist in supply chain management. Knowing when a specific medication delivery device has been activated for the first time may give a manufacturer important supply chain information, for example, how long it takes a medication delivery device to reach a user and be used by a user after the manufacturer has released it for sale. When communicating first use to an external device, the medication delivery device may also communicate its specific identification number to permit a manufacturer to associate the information to a known device and store the information in a database. The information can be categorized by device type, geography, etc.

In some embodiments, the time elapsed from first use of a medication delivery device may be monitored. With some types of medications and medication delivery devices, the medication in a medication delivery device expires after a certain amount of time has elapsed since the medication delivery device was first used to deliver an amount of the medication. This may apply in particular to multi-dose type medication delivery devices. As such, the medication delivery device may detect when the user has actuated the device to deliver medication for the first time. In some embodiments, the device may then begin an internal timer countdown and alert a user that the medication has expired when the timer reaches a predetermined time. Examples of an alert include turning on, off, or blinking a light and/or using a light of a certain color, an auditory sound, a vibration, or any combination thereof. In some embodiments, the medication delivery device may prevent the user from actuating the device, e.g. with a physical and/or electrical lockout that makes delivery impossible. In some embodiments, when the medication delivery device detects that the user has actuated the device to deliver medication for the first time, the medication delivery device may communicate to an external device that first time delivery has occurred. The external device may then begin a countdown to the expiration of the medication. When the countdown as completed, the external device may send an alert to the user and/or communicate to the medication delivery device that the medication has expired.

According to one aspect, a temperature of the medication may be monitored. Medication temperature may be monitored directly or indirectly. One example of direct measurement includes placing a temperature sensor in actual contact with the medication. One example of indirect measurement includes using a temperature sensor to measure the temperature of a region or component close to the medication to approximate what the actual temperature of the medication is. For example, in one embodiment, a temperature sensor is located at the PCB of a medication delivery device. Another example of indirect measurement includes directly measuring the temperature of a material within the medication delivery device that behaves similarly to the actual medication when exposed to various temperature environments.

Temperature measurements may occur periodically. Information relating to the measured temperature may be stored within the medication delivery device, may be communicated to an external device each time a measurement occurs or in batches, or any combination thereof.

When the medication delivery device sensors that the measured temperature is outside an acceptable temperature range, referred to herein as a "temperature excursion," a variety of responses may occur. In some embodiments, the medication delivery device alerts the user directly and/or communicates the information to one or more external devices, which may in turn alert the user. The alert may occur in real-time when the temperature excursion is detected, or may occur the next time the user uses the medication delivery device. In some embodiments, when a temperature excursion is detected, the medication delivery device may store and/or communicate to an external device the time and/or date of the temperature excursion, as well as the measured temperature.

According to one aspect, a medication delivery device and/or an external program that communicates with the medication delivery device, such as a mobile device app, may include security features for controlling wireless communication between the medication delivery device and an external device. In some embodiments, the medication delivery device includes a bond management feature that prevents unwanted access to the medication delivery device from third parties. In this bond management feature, a user has already paired their medication delivery device to the user's mobile device, which may be running an app that is specialized for use with the medication delivery device. If a different mobile device tries to connect with the medication delivery device, the user may receive a notification that a third party is attempting to connect with the medication delivery device. The user may grant or deny permission for the third party to connect with the medication delivery device. In some embodiments, this setting may be "remembered" by the app and/or medication delivery device to avoid repeated notifications. In some embodiments, the app may be configured to include a menu that allows a user to change past authorization settings, e.g. to grant access to a previously denied third party mobile device, or to deny access to a previously approved third party mobile device.

According to one aspect, a user may be notified by an external device or by the medication delivery device itself if the medication delivery device is subject to a recall. In some embodiments, the medication delivery device broadcasts its unique identification number to an external device which may communicate with a remote server that has a database that associates recall information with the identification number. The medication delivery device may communicate directly with the remote server itself.

In some embodiments, an external device such as a mobile device alerts a user that the medication delivery device and/or the medication within the device is subject to a recall and should not be used. The alert may take different forms, including a message displayed by an app running on the mobile device, via text message, via SMS, via email, or any combination thereof.

In some embodiments, the medication delivery device may be instructed by the remote server and/or an intermediate external device such as a mobile device to display an alert informing the user that the medication delivery device and/or medication is subject to a recall and should not be used. In some embodiments, the medication delivery device may activate a physical and/or electrical lockout that prevents the device from being used.

According to one aspect, the medication delivery device may be used to detect failure to administer a dose in accordance with the user's prescribed regimen. For example, if a user accidentally administers two doses at once or too close in time, an external device or the medication delivery device itself will inform the user of the error. As other examples, a user may have accidentally or intuitionally skipped a dose, or may have used an incorrect dose.

Detection of these types of errors may give the user an opportunity to take remedial measures. The external device may provide suggestions to the user for remedial measures, may inform the user's healthcare provider, may connect the user to the healthcare provider, or any combination thereof.

In some embodiments, such errors are able to be monitored because the medication delivery device may be able to detect delivery of medication and may be able to detect the dosage that was delivered. The medication delivery device may communicate such information out to an external device.

Either the external delivery device or the medication delivery device itself may then determine whether such administrations were proper. In some embodiments, an external device or the medication delivery device itself may compare timings and dosages of actual administrations against expected administrations. If the actual administrations do not match with the expected administrations, then the external device and/or the medication delivery device may inform the user, e.g. that they have missed a dose, administered too much or too little of a dosage, or any combination thereof. In one illustrative embodiment, a medication delivery device communicates dosage amounts and delivery times to a mobile device. The mobile device then communicates with a remote server to determine whether this actual administration matches with an expected prescribed regimen. If the actual administration does not match with the expected regimen, then the mobile device alerts the user to an administration error. In situations with a missed dose, the remote server may communicate with the mobile device the inform the mobile device that a dosage should have been administered. If the mobile device has not received information from the medication delivery device indicating that the dosage was administered, the mobile device may then send an alert to the user reminding the user to take their medication.

The shown device is a reusable pen-shaped medication injection device, generally designated, which is manually handled by a user to selectively set a dose and then to inject that set dose. Injection devices of this type are well known, and the description of device is merely illustrative as the sensing system can be adapted for use in variously configured medication delivery devices, including differently constructed pen-shaped medication injection devices, differently shaped injection devices, and infusion pump devices. The medication may be any of a type that may be delivered by such a medication delivery device. Device is intended to be illustrative and not limiting as the sensing system described further below may be used in other differently configured devices.

In another embodiment, the medication delivery device includes a switch comprising a conductive pad and a cantilevered arm that is movable relative to the conductive pad. Contact between the cantilevered arm and the conductive pad closes the switch, and lack of contact between the cantilevered arm and the conductive pad opens to switch. In some embodiments, the switch may be used to determine the amount of a dose of medication delivered by the medication delivery device. In another embodiment, the medication delivery device includes a housing, an outlet, and a dose button that is axially translatable relative to the housing to activate a dose dispensing mode in which medication is dispensed out of the outlet. The medication delivery device also includes a printed circuit board and a switch mounted to the printed circuit board. The switch comprises a conductive pad mounted to the printed circuit board and a cantilevered arm having a first curved portion extending from the printed circuit board. The arm transitions from the first curved portion to a second curved portion that is configured to move toward and contact the conductive pad to close the switch and configured to move away and be spaced from the conductive pad to open the switch. A controller is configured to receive a signal from the switch.

To clarify the use of and to hereby provide notice to the public, the phrases "at least one of <A>, <B>, . . . and <N>" or "at least one of <A>, <B>, . . . <N>, or combinations thereof" or "<A>, <B>, . . . and/or <N>" are defined by the Applicant in the broadest sense, superseding any other implied definitions hereinbefore or hereinafter unless expressly asserted by the Applicant to the contrary, to mean one or more elements selected from the group including A, B, . . . and N. In other words, the phrases mean any combination of one or more of the elements A, B, . . . or N including any one element alone or the one element in combination with one or more of the other elements which may also include, in combination, additional elements not listed.

While various embodiments have been described, it will be apparent to those of ordinary skill in the art that many more embodiments and implementations are possible. Accordingly, the embodiments described herein are examples, not the only possible embodiments and implementations. Furthermore, the advantages described above are not necessarily the only advantages, and it is not necessarily expected that all of the described advantages will be achieved with every embodiment.

## Claims

1. A medication delivery device (10) comprising:
a housing (12);
an outlet (21);
a dose button (30) to activate a dose dispensing mode in which medication is dispensed out of the outlet;
a printed circuit board (77);
a protruded dose element (38) that is rotatable relative to the printed circuit board during a dose dispensing event;
a switch (86') mounted to the printed circuit board, the switch comprising a conductive pad (89) coupled to the printed circuit board and a cantilevered arm (210), the cantilevered arm having a first curved portion (212, 222, 232, 246, 256) extending from the printed circuit board and a second curved portion (214, 224, 234, 244, 254) that, in the dose dispensing event, is configured to move toward and contact the conductive pad to change the switch to a first state when an arm portion (216, 226, 236) of the cantilevered arm is in slidable contact with the protruded dose element, and configured to move away and be spaced from the conductive pad to change the switch to a second state; and
a controller configured to receive a signal from the switch.

2. The medication delivery device of claim 1, wherein, when the cantilevered arm is in an unstressed state, a curvature of the second curved portion (214, 224, 234) faces distally away from the printed circuit board.

3. The medication delivery device of claim 2, wherein, when the cantilevered arm is in an unstressed state, a curvature of the second curved portion (224) extends an end (202) of the cantilevered arm to face toward the first curved portion (222).

4. The medication delivery device of claim 2, wherein, when the cantilevered arm is in an unstressed state, a curvature of the second curved portion (214) extends an end (202) of the cantilevered arm to face away from the first curved portion (212).

5. The medication delivery device of claim 1, wherein said arm portion of the cantilevered arm defines a third curved portion (216, 226) of the cantilevered arm, wherein the third curved portion comprises a U-shape connecting the first curved portion to the second curved portion.

6. The medication delivery device of claim 5, wherein, when the cantilevered arm is in an unstressed state, a curvature of the third curved portion (216, 226) transitions the cantilevered arm from pointing in a distal direction to pointing proximally toward the printed circuit board.

7. The medication delivery device of claim 1, wherein said arm portion of the cantilevered arm defines a third curved portion (236) of the cantilevered arm, wherein the second curved portion (234) connects the first curved portion (232) to the third curved portion.

8. The medication delivery device of claim 7, wherein, when the cantilevered arm is in an unstressed state, a curvature of the first curved portion (232) extends the cantilevered arm distally away from the printed circuit board, and a curvature of the second curved portion (234) defines a U-shape such that a bottom of the second curved portion faces proximally toward the printed circuit board.

9. The medication delivery device of claim 1, wherein the cantilevered arm includes a first branch (240, 250) and a second branch (241, 251), the first curved portion (246, 256) defining a part of the first branch, and the second curved portion (244, 254) defining a part of the second branch.

10. The medication delivery device of claim 9, wherein the first branch is radially offset from the second branch.

11. The medication delivery device of claim 10, wherein the first curved portion (246) is curved about a first axis (1246) and the second curved portion (244) is curved about a second axis (1244), wherein the first axis is parallel with the second axis.

12. The medication delivery device of claim 9, wherein the first curved portion (256) is curved about a first axis (1256) and the second curved portion (254) is curved about a second axis (1254), wherein the first axis is not parallel to the second axis.

13. The medication delivery device of claim 12, wherein the second branch (251) forms a C-shape.

14. The medication delivery device of any one of the preceding claims, further comprising a protrusion (204) extending from the printed circuit board, the protrusion being flanked by the first curved portion and the second curved portion when the second curved portion is in contact with the conductive pad, wherein the protrusion prohibits the second curved portion from further movement toward the first curved portion while the cantilevered arm is moved toward a closed position.

15. The medication delivery device of any one of the preceding claims, wherein the protruded dose element includes a series of teeth (102) that are spaced from one another, the protruded dose element being positioned to permit the teeth to slide against the arm portion of the cantilevered arm to move the cantilevered arm between a closed position and an open position as the protruded dose element is rotated.

16. The medication delivery device of any one of the preceding claims, wherein the protruded dose element is rotatable with the dose button in a dose setting mode, wherein a degree of rotation of the protruded dose element during the dose dispensing mode determines an amount of medication to be dispensed out of the outlet.

17. The medication delivery device of any one of the preceding claims, wherein the switch is configured to sense data indicative of an angular displacement of the protruded dose element relative to the dose button during the dose dispensing mode.

18. The medication delivery device of any one of the preceding claims, wherein the cantilevered arm is a single monolithic component.

19. The medication delivery device of any one of the preceding claims, wherein the switch comprises a base (200) connected to the cantilevered arm, the base being mounted to the printed circuit board.

20. The medication delivery device of any one of the preceding claims, wherein the housing includes a reservoir having a medication.

## Patentansprüche

1. Medikamentenverabreichungsvorrichtung (10), umfassend:
ein Gehäuse (12);
einen Auslass (21);
einen Dosisknopf (30), um einen Dosisabgabemodus, in dem das Medikament aus dem Auslass abgegeben wird, zu aktivieren;
eine Leiterplatte (77);
ein vorstehendes Dosiselement (38), das während eines Dosisabgabeereignisses relativ zu der Leiterplatte drehbar ist;
einen Schalter (86'), der an der Leiterplatte montiert ist, der Schalter umfassend ein leitfähiges Pad (89), das mit der Leiterplatte gekoppelt ist, und einen freitragenden Arm (210), wobei der freitragende Arm einen ersten gekrümmten Abschnitt (212, 222, 232, 246, 256), der sich von der Leiterplatte erstreckt, und einen zweiten gekrümmten Abschnitt (214, 224, 234, 244, 254), der, bei dem Dosisabgabeereignis, konfiguriert ist, um sich zu dem leitfähigen Pad hin zu bewegen und dieses zu kontaktieren, um den Schalter in einen ersten Zustand umzustellen, wenn ein Armabschnitt (216, 226, 236) des freitragenden Arms in gleitbarem Kontakt mit dem vorstehenden Dosiselement steht, und konfiguriert ist, um sich von dem leitfähigen Pad weg zu bewegen und von diesem beabstandet zu sein, um den Schalter in einen zweiten Zustand umzustellen; und
eine Steuerung, die konfiguriert ist, um ein Signal von dem Schalter zu empfangen.

2. Medikamentenverabreichungsvorrichtung nach Anspruch 1, wobei, wenn sich der freitragende Arm in einem unbelasteten Zustand befindet, eine Krümmung des zweiten gekrümmten Abschnitts (214, 224, 234) von der Leiterplatte distal abgewandt ist.

3. Medikamentenverabreichungsvorrichtung nach Anspruch 2, wobei, wenn sich der freitragende Arm in einem unbelasteten Zustand befindet, eine Krümmung des zweiten gekrümmten Abschnitts (224) ein Ende (202) des freitragenden Arms verlängert, um dem ersten gekrümmten Abschnitt (222) zugewandt zu sein.

4. Medikamentenverabreichungsvorrichtung nach Anspruch 2, wobei, wenn sich der freitragende Arm in einem unbelasteten Zustand befindet, eine Krümmung des zweiten gekrümmten Abschnitts (214) ein Ende (202) des freitragenden Arms verlängert, um von dem ersten gekrümmten Abschnitt (212) abgewandt zu sein.

5. Medikamentenverabreichungsvorrichtung nach Anspruch 1, wobei der Armabschnitt des freitragenden Arms einen dritten gekrümmten Abschnitt (216, 226) des freitragenden Arms definiert, wobei der dritte gekrümmte Abschnitt eine U-Form, die den ersten gekrümmten Abschnitt mit dem zweiten gekrümmten Abschnitt verbindet, aufweist.

6. Medikamentenverabreichungsvorrichtung nach Anspruch 5, wobei, wenn sich der freitragende Arm in einem unbelasteten Zustand befindet, eine Krümmung des dritten gekrümmten Abschnitts (216, 226) den freitragenden Arm von einem in eine distale Richtung Weisen zu einem proximalen Weisen zu der Leiterplatte hin übergehen lässt.

7. Medikamentenverabreichungsvorrichtung nach Anspruch 1, wobei der Armabschnitt des freitragenden Arms einen dritten gekrümmten Abschnitt (236) des freitragenden Arms definiert, wobei der zweite gekrümmte Abschnitt (234) den ersten gekrümmten Abschnitt (232) mit dem dritten gekrümmten Abschnitt verbindet.

8. Medikamentenverabreichungsvorrichtung nach Anspruch 7, wobei, wenn sich der freitragende Arm in einem unbelasteten Zustand befindet, eine Krümmung des ersten gekrümmten Abschnitts (232) den freitragende Arm distal von der Leiterplatte weg verlängert und eine Krümmung des zweiten gekrümmten Abschnitts (234) eine U-Form derart definiert, dass eine Unterseite des zweiten gekrümmten Abschnitts der Leiterplatte proximal zugewandt ist.

9. Medikamentenverabreichungsvorrichtung nach Anspruch 1, wobei der freitragende Arm eine erste Abzweigung (240, 250) und eine zweite Abzweigung (241, 251) einschließt, wobei der erste gekrümmte Abschnitt (246, 256) einen Teil der ersten Abzweigung definiert und der zweite gekrümmte Abschnitt (244, 254) einen Teil der zweiten Abzweigung definiert.

10. Medikamentenverabreichungsvorrichtung nach Anspruch 9, wobei die erste Abzweigung von der zweiten Abzweigung radial versetzt ist.

11. Medikamentenverabreichungsvorrichtung nach Anspruch 10, wobei der erste gekrümmte Abschnitt (246) um eine erste Achse (1246) herum gekrümmt ist und der zweite gekrümmte Abschnitt (244) um eine zweite Achse (1244) herum gekrümmt ist, wobei die erste Achse parallel zu der zweiten Achse ist.

12. Medikamentenverabreichungsvorrichtung nach Anspruch 9, wobei der erste gekrümmte Abschnitt (256) um eine erste Achse (1256) herum gekrümmt ist und der zweite gekrümmte Abschnitt (254) um eine zweite Achse (1254) herum gekrümmt ist, wobei die erste Achse nicht parallel zu der zweiten Achse ist.

13. Medikamentenverabreichungsvorrichtung nach Anspruch 12, wobei die zweite Abzweigung (251) eine C-Gestalt formt.

14. Medikamentenverabreichungsvorrichtung nach einem der vorstehenden Ansprüche, ferner umfassend einen Vorsprung (204), der sich von der Leiterplatte erstreckt, wobei der Vorsprung durch den ersten gekrümmten Abschnitt und den zweiten gekrümmten Abschnitt flankiert wird, wenn der zweite gekrümmte Abschnitt mit dem leitfähigen Pad in Kontakt steht, wobei der Vorsprung den zweiten gekrümmten Abschnitt an einer weiteren Bewegung zu dem ersten gekrümmten Abschnitts hin hindert, solange der freitragende Arm zu einer geschlossenen Position hin bewegt wird.

15. Medikamentenverabreichungsvorrichtung nach einem der vorstehenden Ansprüche, wobei das vorstehende Dosiselement eine Reihe von Zähnen (102) einschließt, die voneinander beabstandet sind, wobei das vorstehende Dosiselement positioniert ist, um den Zähnen zu ermöglichen, gegen den Armabschnitt des freitragenden Arms zu gleiten, um den freitragenden Arm zwischen einer geschlossenen Position und einer offenen Position zu bewegen, während das vorstehende Dosiselement gedreht wird.

16. Medikamentenverabreichungsvorrichtung nach einem der vorstehenden Ansprüche, wobei das vorstehende Dosiselement in einem Dosiseinstellmodus mit dem Dosisknopf drehbar ist, wobei ein Drehgrad des vorstehenden Dosiselements während des Dosisabgabemodus eine aus dem Auslass abzugebende Medikamentenmenge bestimmt.

17. Medikamentenverabreichungsvorrichtung nach einem der vorstehenden Ansprüche, wobei der Schalter konfiguriert ist, um Daten zu erfassen, die eine Winkelverschiebung des vorstehenden Dosiselements relativ zu dem Dosisknopf während des Dosisabgabemodus angibt.

18. Medikamentenverabreichungsvorrichtung nach einem der vorstehenden Ansprüche, wobei der freitragende Arm eine einzelne monolithische Komponente ist.

19. Medikamentenverabreichungsvorrichtung nach einem der vorstehenden Ansprüche, wobei der Schalter eine Basis (200), die mit dem freitragenden Arm verbunden ist, umfasst, wobei die Basis an der Leiterplatte montiert ist.

20. Medikamentenverabreichungsvorrichtung nach einem der vorstehenden Ansprüche, wobei das Gehäuse einen Vorratsbehälter, der ein Medikament aufweist, einschließt.

## Revendications

1. Dispositif d'administration de médicament (10) comprenant :
un boîtier (12) ;
une sortie (21) ;
un bouton de dose (30) pour activer un mode de distribution de dose dans lequel le médicament est distribué par la sortie ;
une carte de circuit imprimé (77) ;
un élément de dose en saillie (38) qui peut être en rotation par rapport à la carte de circuit imprimé pendant un événement de distribution de dose ;
un commutateur (86') monté sur la carte de circuit imprimé, le commutateur comprenant un plot conducteur (89) couplé à la carte de circuit imprimé et un bras en porte-à-faux (210), le bras en porte-à-faux ayant une première partie courbée (212, 222, 232, 246, 256) s'étendant à partir de la carte de circuit imprimé et une deuxième partie courbée (214, 224, 234, 244, 254) qui, dans l'événement de distribution de dose, est conçue pour se rapprocher et entrer en contact avec le plot conducteur afin de commuter le commutateur vers un premier état lorsqu'une partie de bras (216, 226, 236) du bras en porte-à-faux est en contact coulissant avec l'élément de dose en saillie, et conçue pour s'éloigner et être espacée du plot conducteur afin de changer le commutateur vers un second état ; et
un dispositif de commande configuré pour recevoir un signal à partir du commutateur.

2. Dispositif d'administration de médicament selon la revendication 1, dans lequel, lorsque le bras en porte-à-faux est dans un état non contraint, une courbure de la deuxième partie courbée (214, 224, 234) est orientée de manière distale à l'écart de la carte de circuit imprimé.

3. Dispositif d'administration de médicament selon la revendication 2, dans lequel, lorsque le bras en porte-à-faux est dans un état non contraint, une courbure de la deuxième partie courbée (224) étend une extrémité (202) du bras en porte-à-faux pour l'orienter vers la première partie courbée (222).

4. Dispositif d'administration de médicament selon la revendication 2, dans lequel, lorsque le bras en porte-à-faux est dans un état non contraint, une courbure de la deuxième partie courbée (214) étend une extrémité (202) du bras en porte-à-faux pour l'orienter à l'écart de la première partie courbée (212).

5. Dispositif d'administration de médicament selon la revendication 1, dans lequel ladite partie de bras du bras en porte-à-faux définit une troisième partie courbée (216, 226) du bras en porte-à-faux, dans lequel la troisième partie courbée comprend une forme en U reliant la première partie courbée à la deuxième partie courbée.

6. Dispositif d'administration de médicament selon la revendication 5, dans lequel, lorsque le bras en porte-à-faux est dans un état non contraint, une courbure de la troisième partie courbée (216, 226) fait passer le bras en porte-à-faux d'un pointage dans une direction distale à un pointage de manière proximale vers la carte de circuit imprimé.

7. Dispositif d'administration de médicament selon la revendication 1, dans lequel ladite partie de bras du bras en porte-à-faux définit une troisième partie courbée (236) du bras en porte-à-faux, dans lequel la deuxième partie courbée (234) relie la première partie courbée (232) à la troisième partie courbée.

8. Dispositif d'administration de médicament selon la revendication 7, dans lequel, lorsque le bras en porte-à-faux est dans un état non contraint, une courbure de la première partie courbée (232) étend le bras en porte-à-faux de manière distale à l'écart de la carte de circuit imprimé, et une courbure de la deuxième partie courbée (234) définit une forme en U de telle sorte qu'un fond de la deuxième partie courbée est orienté de manière proximale vers la carte de circuit imprimé.

9. Dispositif d'administration de médicament selon la revendication 1, dans lequel le bras en porte-à-faux comporte une première branche (240, 250) et une seconde branche (241, 251), la première partie courbée (246, 256) définissant une part de la première branche, et la deuxième partie courbée (244, 254) définissant une part de la seconde branche.

10. Dispositif d'administration de médicament selon la revendication 9, dans lequel la première branche est décalée radialement par rapport à la seconde branche.

11. Dispositif d'administration de médicament selon la revendication 10, dans lequel la première partie courbée (246) est courbée autour d'un premier axe (1246) et la deuxième partie courbée (244) est courbée autour d'un second axe (1244), dans lequel le premier axe est parallèle au second axe.

12. Dispositif d'administration de médicament selon la revendication 9, dans lequel la première partie courbée (256) est courbée autour d'un premier axe (1256) et la deuxième partie courbée (254) est courbée autour d'un second axe (1254), dans lequel le premier axe n'est pas parallèle au second axe.

13. Dispositif d'administration de médicament selon la revendication 12, dans lequel la seconde branche (251) forme un C.

14. Dispositif d'administration de médicament selon l'une quelconque des revendications précédentes, comprenant en outre une saillie (204) s'étendant à partir de la carte de circuit imprimé, la saillie étant flanquée de la première partie courbée et de la deuxième partie courbée lorsque la deuxième partie courbée est en contact avec le plot conducteur, dans lequel la saillie empêche la deuxième partie courbée de se déplacer davantage vers la première partie courbée lorsque le bras en porte-à-faux est déplacé vers une position fermée.

15. Dispositif d'administration de médicament selon l'une quelconque des revendications précédentes, dans lequel l'élément de dose en saillie comporte une série de dents (102) qui sont espacées les unes des autres, l'élément de dose en saillie étant positionné de manière à permettre aux dents de glisser contre la partie de bras du bras en porte-à-faux pour déplacer le bras en porte-à-faux entre une position fermée et une position ouverte lors de la rotation de l'élément de dose en saillie.

16. Dispositif d'administration de médicament selon l'une quelconque des revendications précédentes, dans lequel l'élément de dose en saillie est rotatif avec le bouton de dose dans un mode de réglage de dose, dans lequel un degré de rotation de l'élément de dose en saillie pendant le mode de distribution de dose détermine une quantité de médicament à distribuer par la sortie.

17. Dispositif d'administration de médicament selon l'une quelconque des revendications précédentes, dans lequel le commutateur est configuré pour détecter des données indiquant un déplacement angulaire de l'élément de dose en saillie par rapport au bouton de dose pendant le mode de distribution de dose.

18. Dispositif d'administration de médicament selon l'une quelconque des revendications précédentes, dans lequel le bras en porte-à-faux est un composant monolithique unique.

19. Dispositif d'administration de médicament selon l'une quelconque des revendications précédentes, dans lequel le commutateur comprend une base (200) reliée au bras en porte-à-faux, la base étant montée sur la carte de circuit imprimé.

20. Dispositif d'administration de médicament selon l'une quelconque des revendications précédentes, dans lequel le boîtier comporte un réservoir ayant un médicament.
